# EUROPÄISCHE PATENTSCHRIFT

(11) **EP 1 068 301 B1**
(45) Veröffentlichungstag und Bekanntmachung des Hinweises auf die Patenterteilung: **26.07.2006**
(21) Anmeldenummer: 99917952.6
(22) Anmeldetag: 01.04.1999
(51) Int. Cl.: C12N 9/10, C12N 15/54, C12P 21/00

(54) **BAKTERIELLE TRANSGLUTAMINASEN**
BACTERIAL TRANSGLUTAMINASES
TRANSGLUTAMINASES BACTERIENNES

(30) Priorität: 02.04.1998 DE 19814860
(43) Veröffentlichungstag der Anmeldung: 17.01.2001
(73) Patentinhaber: N-Zyme BioTec GmbH, 64295 Darmstadt (DE)
(72) Erfinder: FUCHSBAUER, Hans-Lothar, D-64367 Mühltal (DE); PASTERNACK, Ralf, D-64347 Griesheim (DE); DORSCH, Simone, D-64291 Darmstadt (DE); OTTERBACH, Jens, D-69221 Dossenheim (DE); ROBENEK, Isabella, D-64625 Bensheim (DE); MAINUSCH, Martina, D-64380 Ro dorf (DE); DAUSCHER, Christine, D-64347 Griesheim (DE)
(74) Vertreter: Arth, Hans-Lothar
(86) Internationale Anmeldenummer: PCT/EP1999/002259
(87) Internationale Veröffentlichungsnummer: WO 1999/051723

(56) Entgegenhaltungen:
- EP-A- 0 481 504
- EP-A- 0 726 317
- WO-A-96/06931
- WASHIZU, K. ET AL.: "Molecular cloning of the gene for microbial transglutaminase from Streptoverticillium and its expression in Streptomyces lividans" BIOSCI. BIOTECH. BIOCHIM., Bd. 58, Nr. 1, 1994, Seiten 82-87, XP002117287
- KAWAI, M. ET AL.: "High-level expression of chemically synthesized gene for microbial transglutaminase from Streptoverticillium in Escherichia coli" BIOSCI. BIOTECH. BIOCHEM., Bd. 61, Nr. 5, 1997, Seiten 830-835, XP002117288
- BISHOP, P.D. ET AL.: "Expression, purification, and characterization of human factor XIII in Saccharomyces cerevisiae" BIOCHEMISTRY, Bd. 29, 1990, Seiten 1861-1869, XP000567873
- DATABASE WPI Derwent Publications Ltd., London, GB; AN 1994-079294 XP002127090 & JP 06 030771 A (AJINOMOTO), 8. Februar 1994 (1994-02-08)
- PASTERNACK, R. ET AL.: "Bacterial pro-transglutaminase from Streptoverticillium mobarense ..." EUR. J. BIOCHEM., Bd. 257, 1998, Seiten 570-576, XP002127089
- KIKUCHI Y. ET AL: "Secretion of Active-Form Streptoverticillium mobaraense Transglutaminase by Corynebacterium glutamicum: Processing of the Pro-Transglutaminase by a Cosecreted Subtilisin-Like Protease from Streptomyces albogriseolus" APPLIED AND ENVIRONMENTAL MICROBIOLOGY, Bd. 69, Nr. 1, Januar 2003 (2003-01), Seiten 358-366,

### SEQUENZPROTOKOLL

(1) ALLGEMEINE ANGABEN:
   (i) ANMELDER:
      (A) NAME: Prof. Dr. Hans-Lothar Fuchsbauer
      (B) STRASSE: Füerthweg la
      (C) ORT: Mühltal
      (E) LAND: Deutschland
      (F) POSTLEITZAHL: 64367
   (ii) BEZEICHNUNG DER ERFINDUNG: Bakterielle Transglutaminasen
   (iii) ANZAHL DER SEQUENZEN: 19
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 331 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 1:
   Asp Ser Asp Asp Arg Val Thr Pro Pro Ala Glu Pro Leu Asp Arg Met
   1               5                   10                  15
   Pro Asp Pro Tyr Arg Pro Ser Tyr Gly Arg Ala Glu Thr Val Val Asn
               20                  25                  30
   Asn Tyr Ile Arg Lys Trp Gln Gln Val Tyr Ser His Arg Asp Gly Arg
           35                  40                  45
   Lys Gln Gln Met Thr Glu Glu Gln Arg Glu Trp Leu Ser Tyr Gly Cys
       50                  55                  60
   Val Gly Val Thr Trp Val Asn Ser Gly Gln Tyr Pro Thr Asn Arg Leu
   65                  70                  75                  80
   Ala Phe Ala Ser Phe Asp Glu Asp Arg Phe Lys Asn Glu Leu Lys Asn
                   85                  90                  95
   Gly Arg Pro Arg Ser Gly Glu Thr Arg Ala Glu Phe Glu Gly Arg Val
               100                 105                 110
   Ala Lys Glu Ser Phe Asp Glu Glu Lys Gly Phe Gln Arg Ala Arg Glu
           115                 120                 125
   Val Ala Ser Val Met Asn Arg Ala Leu Glu Asn Ala His Asp Glu Ser
       130                 135                 140
   Ala Tyr Leu Asp Asn Leu Lys Lys Glu Leu Ala Asn Gly Asn Asp Ala
   145                 150                 155                 160
   Leu Arg Asn Glu Asp Ala Arg Ser Pro Phe Tyr Ser Ala Leu Arg Asn
                   165                 170                 175
   Thr Pro Ser Phe Lys Glu Arg Asn Gly Gly Asn His Asp Pro Ser Arg
               180                 185                 190
   Met Lys Ala Val Ile Tyr Ser Lys His Phe Trp Ser Gly Gln Asp Arg
           195                 200                 205
   Ser Ser Ser Ala Asp Lys Arg Lys Tyr Gly Asp Pro Asp Ala Phe Arg
       210                 215                 220
   Pro Ala Pro Gly Thr Gly Leu Val Asp Met Ser Arg Asp Arg Asn Ile
   225                 230                 235                 240
   Pro Arg Ser Pro Thr Ser Pro Gly Glu Gly Phe Val Asn Phe Asp Tyr
                   245                 250                 255
   Gly Trp Phe Gly Ala Gln Thr Glu Ala Asp Ala Asp Lys Thr Val Trp
               260                 265                 270
   Thr His Gly Asn His Tyr His Ala Pro Asn Gly Ser Leu Gly Ala Met
           275                 280                 285
   His Val Tyr Glu Ser Lys Phe Arg Asn Trp Ser Glu Gly Tyr Ser Asp
       290                 295                 300
   Phe Asp Arg Gly Ala Tyr Val Ile Thr Phe Ile Pro Lys Ser Trp Asn
   305                 310                 315                 320
   Thr Ala Pro Asp Lys Val Lys Gln Gly Trp Pro
                   325                 330
(2) ANGABEN ZU SEQ ID NO: 2:
   (i) SEQUENZKENNZEICHEN:
      (A) LÄNGE: 45 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 2:
   Asp Asn Gly Ala Gly Glu Glu Thr Lys Ser Tyr Ala Glu Thr Tyr Arg
   1 5 10 15
   Leu Thr Ala Asp Asp Val Ala Asn Ile Asn Ala Leu Asn Glu Ser Ala
   20 25 30
   Pro Ala Ala Ser Ser Ala Gly Pro Ser Phe Arg Ala Pro
   35 40 45
(2) ANGABEN ZU SEQ ID NO: 3:
   (i) SEQUENZKENNZEICHEN:
      (A) LÄNGE: 42 Aminosäuren
      (B) ART: Aminosäure
      (C) STRANGFORM: Einzelstrang
      (D) TOPOLOGIE: linear
   (ii) ART DES MOLEKÜLS: Peptid
   (ix) MERKMAL:
      (A) NAME/SCHLÜSSEL: Aminosäuren
      (B) LAGE: 30
      (D) SONSTIGE ANGABEN:/product=" Folge mehrerer nicht charakterisierter Aminosäuren"
         /label=X
   (xi) SEQUENZBESCHREIBUNG: SEQ ID NO: 3:
   Ala Gly Ser Ser Asp Gly Glu Lys Lys Gly Ser Tyr Ala Ala Thr His
   1 5 10 15
   Gly Leu Thr Ala Asp Asp Val Asp Asn Ile Asn Ala Leu Xaa Phe Pro
   20 25 30
   Pro Ser Ala Gly Pro Ser Leu Arg Ala Ala
   35 40
(2) ANGABEN ZU SEQ ID NO: 4:
   (i) SEQUENZKENNZEICHEN:
      (A) LÄNGE: 331 Aminosäuren
      (B) ART: Aminosäure
      (C) STRANGFORM: Einzelstrang
      (D) TOPOLOGIE: linear
   (ii) ART DES MOLEKÜLS: Protein
   (xi) SEQUENZBESCHREIBUNG: SEQ ID NO: 4:
   Asp Ser Asp Asp Arg Val Thr Pro Pro Ala Glu Pro Leu Asp Arg Met
   1               5                   10                  15
   Pro Asp Pro Tyr Arg Pro Ser Tyr Gly Arg Ala Glu Thr Val Val Asn
               20                  25                  30
   Asn Tyr Ile Arg Lys Trp Gln Gln Val Tyr Ser His Arg Asp Gly Arg
           35                  40                  45
   Lys Gln Gln Met Thr Glu Glu Gln Arg Glu Trp Leu Ser Tyr Gly Cys
       50                  55                  60
   Val Gly Val Thr Trp Val Asn Ser Gly Gln Tyr Pro Thr Asn Arg Leu
   65                  70                  75                  80
   Ala Phe Ala Ser Phe Asp Glu Asp Arg Phe Lys Asn Glu Leu Lys Asn
                   85                  90                  95
   Gly Arg Pro Arg Ser Gly Glu Thr Arg Ala Glu Phe Glu Gly Arg Val
               100                 105                 110
   Ala Lys Glu Ser Phe Asp Glu Glu Lys Gly Phe Gln Arg Ala Arg Glu
           115                 120                 125
   Val Ala Ser Val Met Asn Arg Ala Leu Glu Asn Ala His Asp Glu Ser
       130                 135                 140
   Ala Tyr Leu Asp Asn Leu Lys Lys Glu Leu Ala Asn Gly Asn Asp Ala
   145                 150                 155                 160
   Leu Arg Asn Glu Asp Ala Arg Ser Pro Phe Tyr Ser Ala Leu Arg Asn
                   165                 170                 175
   Thr Pro Ser Phe Lys Glu Arg Asn Gly Gly Asn His Asp Pro Ser Arg
               180                 185                 190
   Met Lys Ala Val Ile Tyr Ser Lys His Phe Trp Ser Gly Gln Asp Arg
           195                 200                 205
   Ser Ser Ser Ala Asp Lys Arg Lys Tyr Gly Asp Pro Asp Ala Phe Arg
       210                 215                 220
   Pro Ala Pro Gly Thr Gly Leu Val Asp Met Ser Arg Asp Arg Asn Ile
   225                 230                 235                 240
   Pro Arg Ser Pro Thr Ser Pro Gly Glu Gly Phe Val Asn Phe Asp Tyr
                   245                 250                 255
   Gly Trp Phe Gly Ala Gln Thr Glu Ala Asp Ala Asp Lys Thr Val Trp
               260                 265                 270
   Thr His Gly Asn His Tyr His Ala Pro Asn Gly Ser Leu Gly Ala Met
           275                 280                 285
   His Val Tyr Glu Ser Lys Phe Arg Asn Trp Ser Glu Gly Tyr Ser Asp
       290                 295                 300
   Phe Asp Arg Gly Ala Tyr Val Ile Thr Phe Ile Pro Lys Ser Trp Asn
   305                 310                 315                 320
   Thr Ala Pro Asp Lys Val Lys Gln Gly Trp Pro
                   325                 330
(2) ANGABEN ZU SEQ ID NO: 5:
   (i) SEQUENZKENNZEICHEN:
      (A) LÄNGE: 328 Aminosäuren
      (B) ART: Aminosäure
      (C) STRANGFORM: Einzelstrang
      (D) TOPOLOGIE: linear
   (ii) ART DES MOLEKÜLS: Protein
   (ix) MERKMAL:
      (A) NAME/SCHLÜSSEL: Aminosäuren
      (B) LAGE: 326
      (D) SONSTIGE ANGABEN:/product=" Xaa ist N oder K"
         /label=X1
   (ix) MERKMAL:
      (A) NAME/SCHLÜSSEL: Aminosäuren
      (B) LAGE: 328
      (D) SONSTIGE ANGABEN:/product=" Xaa ist der C-Terminus"
         /label=X2
   (xi) SEQUENZBESCHREIBUNG: SEQ ID NO: 5:
   Ala Phe Asp Asp Asp Arg Val Thr Pro Pro Ala Glu Pro Leu Asp Arg
   1               5                   10                  15
   Met Pro Asp Ala Tyr Arg Ala Tyr Gly Gly Arg Ala Thr Thr Val Ile
               20                  25                  30
   Asn Asn Tyr Ile Arg Lys Trp Gln Gln Val Tyr Ser His Arg Asp Gly
           35                  40                  45
   Lys Ile Arg Gln Met Thr Glu Glu Gln Arg Glu Lys Leu Ser Tyr Gly
       50                  55                  60
   Cys Val Gly Val Thr Trp Ala Asn Ser Gly Pro Tyr Pro Thr Asn Arg
   65                  70                  75                  80
   Leu Ala Phe Ala Ser Phe Asp Glu Asp Lys Tyr Arg Asn Asn Leu Lys
                   85                  90                  95
   Asn Ser Ser Pro Gly Leu Gly Glu Thr Arg Ala Glu Phe Glu Gly Arg
               100                 105                 110
   Ile Ala Lys Ala Ser Phe Asp Glu Arg Ala Gly Phe Lys Arg Ala Arg
           115                 120                 125
   Asp Val Ala Ala Ile Met Asn Lys Ala Leu Glu Asn Ala His Asp Glu
       130                 135                 140
   Gly Thr Tyr Ile Asp His Leu Lys Thr Glu Leu Thr Asn Asn Asn Asp
   145                 150                 155                 160
   Ala Leu Leu Gln Glu Asp Thr Ser Ser Asn Phe Tyr Ser Ala Leu Arg
                   165                 170                 175
   Asp Thr Pro Ser Phe Lys Glu Arg Asp Gly Gly Asn Tyr Asp Pro Ser
               180                 185                 190
   Lys Met Lys Ala Val Ile Tyr Ser Lys His Phe Trp Ser Gly Gln Asp
           195                 200                 205
   Arg Arg Gly Ser Asp Asp Lys Arg Lys Tyr Gly Asp Pro Glu Ala Phe
       210                 215                 220
   Arg Pro Asp Gln Arg Thr Gly Leu Val Asp Met Ser Lys Asp Arg Asn
   225                 230                 235                 240
   Ile Pro Arg Ser Pro Ala Arg Ser Gly Glu Ser Trp Val Asn Phe Asp
                   245                 250                 255
   Tyr Gly Trp Phe Gly Ala Gln Thr Glu Ala Asp Pro Gly Gln Thr Ile
   260 265 270
   Trp Thr His Gly Asp His Tyr His Ala Pro Asn Gly Ser Leu Gly Pro
   275 280 285
   Met His Val Tyr Glu Ser Lys Phe Gln Asn Trp Ser Ala Gly Tyr Glu
   290 295 300
   Asp Phe Asp Arg Gly Thr Tyr Val Ile Thr Phe Ile Pro Lys Ser Leu
   305 310 315 320
   Asn Thr Ala Pro Ala Xaa Val Xaa
   325
(2) ANGABEN ZU SEQ ID NO: 6:
   (i) SEQUENZKENNZEICHEN:
      (A) LÄNGE: 5 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 6:
   Lys Gln Gly Trp Pro
   1 5
(2) ANGABEN ZU SEQ ID NO: 7:
   (i) SEQUENZKENNZEICHEN:
      (A) LÄNGE: 245 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 7:
   CAAAGGAGTT GCAGGTTTCC ATGCGCTATA CGCCGGAGGC TCTCGTCTTC GCCACTATGA 60
   GTGCGGTTTA TGCACCGCCG GATTCATGCC GTCGGCCGGC GAGGCCGCCG CCGACAATGG 120
   CGCGGGGGAA GAGACGAAGT CCTACGCCGA AACCTACCGC CTCACGGCGG ATGACGTCGC 180
   GACATCAACG CGCTCAACGA AGCGCTCCGG CCGCTTCGAG CGCCGGCCCG TCGTTCCGGG 240
   CCCCC 245
(2) ANGABEN ZU SEQ ID NO: 8:
   (i) SEQUENZKENNZEICHEN:
      (A) LÄNGE: 996 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 8:
   GACTCCGACG ACAGGGTCAC CCCTCCCGCC GAGCCGCTCG ACAGGATGCC CGACCCGTAC    60
   CGTCCCTCGT ACGGCAGGGC CGAGACGGTC GTCAACAACT ACATACGCAA GTGGCAGCAG   120
   GTCTACAGCC ACCGCGACGG CAGGAAGCAG CAGATGACCG AGGAGCAACG GGAGTGGCTG   180
   TCCTACGGCT GCGTCGGTGT CACCTGGGTC AATTCGGGTC AGTACCCCAC GAACAGACTG   240
   GCCTTCGCGT CCTTCGACGA GGACAGGTTC AAGAACGAGC TGAAGAACGG CAGGCCCCGG   300
   TCCGGCGAGA CGCGGGCGGA GTTCGAGGGC CGCGTCGCGA AGGAGAGCTT TGATGAAGAG   360
   AAGGGGTTCC AGCGGGCGCG TGAGGTGGCG TCCGTGATGA ACAGGGCCCT GGAGAACGCC   420
   CACGACGAGA GCGCTTACCT CGACAACCTC AAGAAGGAAC TGGCGAACGG CAACGACGCC   480
   CTGCGCAACG AGGACGCCCG TTCCCCGTTC TACTCGGCGC TGCGGAACAC GCCGTCCTTT   540
   AAGGAGCGGA ACGGAGGCAA TCACGACCCG TCCAGGATGA AGGCCGTCAT CTACTCGAAG   600
   CACTTCTGGA GCGGCCAGGA CCGGTCGAGT TCGGCCGACA AGAGGAAGTA CGGCGACCCG   660
   GACGCTTTCC GCCCGGCCCC CGGGACCGGC CTGGTCGACA TGTCGAGGGA CAGGAACATT   720
   CCGCGCAGCC CCACCAGCCC CGGTGAGGGA TTCGTCAATT TCGACTACGG CTGGTTCGGC   780
   GCCCAGACGG AAGCGGACGC CGACAAGACC GTCTGGACCC ACGGAAATCA CTATCACGCG   840
   CCCAATGGCA GCCTTGGTGC CATGCATGTA TACGAGAGCA AGTTCCGCAA CTGGTCCGAA   900
   GGTTACTCCG ACTTCGACCG CGGAGCCTAT GTGATCACCT TCATCCCCAA GAGCTGGAAC   960
   ACCGCCCCCG ACAAGGTAAA GCAGGGCTGG CCGTGA                             996
(2) ANGABEN ZU SEQ ID NO: 9:
   (i) SEQUENZKENNZEICHEN:
      (A) LÄNGE: 135 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREI9UNG: SEQ ID NO: 9:
   GACAATGGCG CGGGGGAAGA GACGAAGTCC TACGCCGAAA CCTACCGCCT CACGGCGGAT 60
   GACGTCGCGA ACATCAACGC GCTCAACGAA AGCGCTCCGG CCGCTTCGAG CGCCGGCCCG 120
   TCGTTCCGGG CCCCC 135
(2) ANGABEN ZU SEQ ID NO: 10:
   (i) SEQUENZKENNZEICHEN:
      (A) LÄNGE: 37 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (ix) MERKMAL:
      (A) NAME/SCHLÜSSEL: Nucleotidfolge
      (3) LAGE: 1
      (D) SONSTIGE ANGABEN:/product=" N ist eine unbestimmte Anzahl an Nucleotiden einschließlich eines N-terminalen ATG-Codons" /label=Y1
   (xi) SEQUENZBESCHREIBUNG: SEQ ID NO: 10: NTTCCCGCCG AGCGCCGGCC CGTCCCTCCG GGCCGCC 37
(2) ANGABEN ZU SEQ ID NO: 11:
   (i) SEQUENZKENNZEICHEN:
      (A) LÄNGE: 996 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 11:
   GACTCCGACG ACAGGGTCAC CCCTCCCGCC GAGCCGCTCG ACAGGATGCC CGACCCGTAC    60
   CGTCCCTCGT ACGGCAGGGC CGAGACGGTC GTCAACAACT ACATACGCAA GTGGCAGCAG   120
   GTCTACAGCC ACCGCGACGG CAGGAAGCAG CAGATGACCG AGGAGCAGCG GGAGTGGCTG   180
   TCCTACGGCT GCGTCGGTGT CACCTGGGTC AATTCGGGTC AGTACCCGAC GAACAGACTG   240
   GCCTTCGCGT CCTTCGACGA GGACAGGTTC AAGAACGAGC TGAAGAACGG CAGGCCCCGG   300
   TCCGGCGAGA CGCGGGCGGA GTTCGAGGGC CGCGTCGCGA AGGAGAGCTT CGACGAGGAG   360
   AAGGGCTTCC AGCGGGCGCG TGAGGTGGCG TCCGTCATGA ACAGGGCCCT GGAGAACGCC   420
   CACGACGAGA GCGCTTACCT CGACAACCTC AAGAAGGAAC TGGCGAACGG CAACGACGCC   480
   CTGCGCAACG AGGACGCCCG TTCCCCGTTC TACTCGGCGC TGCGGAACAC GCCGTCCTTC   540
   AAGGAGCGGA ACGGAGGCAA TCACGACCCG TCCAGGATGA AGGCCGTCAT CTACTCGAAG   600
   CACTTCTGGA GCGGCCAGGA CCGGTCGAGT TCGGCCGACA AGAGGAAGTA CGGCGACCCG   660
   GACGCCTTCC GCCCCGCCCC GGGCACCGGC CTGGTCGACA TGTCGAGGGA CAGGAACATT   720
   CCGCGCAGCC CCACCAGCCC CGGTGAGGGA TTCGTCAATT TCGACTACGG CTGGTTCGGC   780
   GCCCAGACGG AAGCGGACGC CGACAAGACC GTCTGGACCC ACGGAAATCA CTATCACGCG   840
   CCCAATGGCA GCCTGGGTGC CATGCATGTC TACGAGAGCA AGTTCCGCAA CTGGTCCGAG   900
   GGTTACTCGG ACTTCGACCG CGGAGCCTAT GTGATCACCT TCATCCCCAA GAGCTGGAAC   960
   ACCGCCCCCG ACAAGGTAAA GCAGGGCTGG CCGTGA                             996
(2) ANGABEN ZU SEQ ID NO: 12:
   (i) SEQUENZKENNZEICHEN:
      (A) LÄNGE: 982 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (ix) MERKMAL:
      (A) NAME/SCHLÜSSEL: Nucleotid
      (B) LAGE: 978
      (D) SONSTIGE ANGABEN:/product=" N ist ein nicht bestimmtes Nucleotid" /label=Y2
   (ix) MERKMAL:
      (A) NAME/SCHLÜSSEL: Nucleotid
      (B) LAGE: 981
      (D) SONSTIGE ANGABEN:/product=" N ist ein nicht bestimmtes Nucleotid" /label=Y3
   (ix) MERKMAL:
      (A) NAME/SCHLÜSSEL: Nucleotidfelge
      (B) LAGE: 982
      (D) SONSTIGE ANGABEN:/product=" N ist das 3'-Ende der kodierenden Nucleinsäuresequenz" /label=Y4
   (xi) SEQUENZBESCHREIBUNG: SEQ ID NO: 12:
   GCCTTCGACG ACGACCGGGT GACCCCTCCC GCCGAGCCGC TCGACAGGAT GCCCGACGCG 60
   TACCGGGCCT ACGGAGGCAG GGCCACCACG GTCATCAACA ACTACATACG CAAGTGGCAG 120
   CAGGTCTACA GCCACCGCGA CGGCAAGATA CGACAGATGA CCGAAGAGCA GCGGGAGAAG 180
   CTGTCCTACG GTTGCGTCGG CGTCACCTGG GCCAATTCGG GCCCCTACCC GACGAACAGA 240
   CTGGCGTTCG CGTCCTTCGA CGAGGACAAG TACAGGAACA ACCTCAAAAA CAGCAGCCCA 300
   GGCCTCGGTG AAACGCGGGC GGAGTTCGAG GGCCGCATCG CCAAGGCCAG CTTCGACGAG 360
   CGGGCGGGCT TCAAGCGGGC GCGTGACGTG GCGGCCATCA TGAACAAGGC CCTGGAGAAC 420
   GCCCACGACG AGGGCACGTA CATCGACCAC CTCAAGACAG AGCTCACGAA CAACAACGAC 480
   GCTCTGCTCC AGGAGGACAC CAGCTCGAAC TTCTACTCGG CGCTGAGGGA CACACCATCC 540
   TTCAAGGAGA GAGACGGAGG CAACTACGAC CCGTCGAAGA TGAAGGCGGT GATCTACTCG 600
   AAGCACTTCT GGAGCGGGCA GGACCGGCGC GGCTCCGACG ACAAGAGGAA GTACGGCGAC 660
   CCGGAAGCCT TCCGGCCCGA CCAGCGGACC GGTCTGGTCG ACATGTCGAA GGACAGAAAC 720
   ATCCCGCGCA GCCCCGCCCG TTCCGGTGAA AGCTGGGTCA ACTTCGACTA CGGCTGGTTC 780
   GGTGCGCAGA CAGAGGCGGA CCCCGGGCAA ACCATATGGA CCCACGGCGA CCACTATCAC 840
   GCGCCCAACG GCAGCCTGGG CCCCATGCAT GTATACGAGA GCAAGTTCCA GAACTGGTCG 900
   GCCGGGTACG AGGACTTCGA TCGCGGAACC TACGTGATCA CGTTCATACC CAAGAGCTTG 960
   AACACCGCCC CCGCCAANGT NN 982
(2) ANGABEN ZU SEQ ID NO: 13:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: sonstige Nucleinsäure
   (xi) SEQUENZBESCHREIBUNG: SEQ ID NO: 13:
      AAGCAGGGCT GGCCGTGA 18
(2) ANGABEN ZU SEQ ID NO: 14:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 14:
      GACAATGGCG CGGGGGAA 18
(2) ANGABEN ZU SEQ ID NO: 15:
   (i) SEQUENZKENNZEICHEN:
      (A) LÄNGE: 18 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 15:
      GCCGGCAGCA GCGACGGG 18
(2) ANGABEN ZU SEQ ID NO: 16:
   (i) SEQUENZKENNZEICHEN:
      (A) LÄNGE: 18 Basenpaare
      (B). ART: Nucleotid
      (C) STRANGFORM: Einzelstrang
      (D) TOPOLOGIE: linear
   (ii) ART DES MOLEKÜLS: Sonstige Nucleinsäure
   (xi) SEQUENZBESCHREIBUNG: SEQ ID NO: 16:
      TCACGGCCAG CCCTGCTT 18
(2) ANGABEN ZU SEQ ID NO: 17:
   (i) SEQUENZKENNZEICHEN:
      (A) LÄNGE: 6 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 17:
   Asp Asn Gly Ala Gly Glu
   1               5
(2) ANGABEN ZU SEQ ID NO: 18:
   (i) SEQUENZKENNZEICHEN:
      (A) LÄNGE: 6 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 18:
   Ala Gly Ser Ser Asp Gly
   1               5
(2) ANGABEN ZU SEQ ID NO: 19:
   (i) SEQUENZKENNZEICHEN:
      (A) LÄNGE: 5 Aminosäuren
      (B) ART: Aminosäure
      (C) STRANGFORM: Einzelstrang
      (D) TOPOLOGIE: linear
   (ii) ART DES MOLEKÜLS: Peptid
   (xi) SEQUENZBESCHREIBUNG: SEQ ID NO: 19:
   Lys Gln Gly Trp Pro
   1               5

## Beschreibung

Die vorliegende Erfindung bezieht sich auf Transglutaminase-Polypeptidmoleküle, Transglutaminase-Nukleinsäuremoleküle, Expressionsvektoren und Wirtsorganismen sowie Verfahren zum Herstellen eines inaktiven "Pro"-Polypeptidmoleküls und zum Aktivieren desselben. Weiterhin werden Antikörper gegen die erfindungsgemäßen Transglutaminase-Polypeptidmoleküle zur Verfügung gestellt.

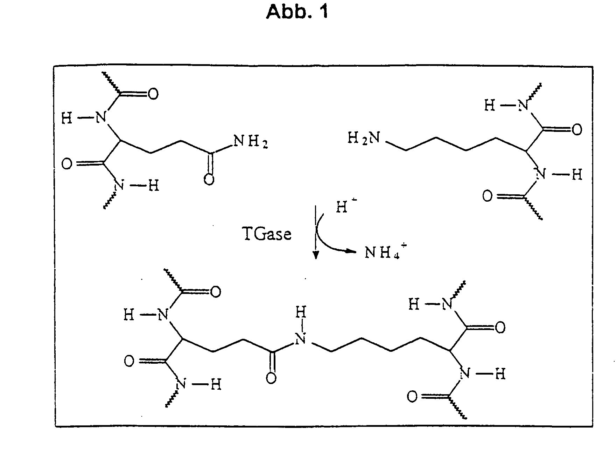

Die ubiquitär auftretende Enzymfamilie der Transglutaminasen katalysiert die kovalente Verknüpfung von Seitenketten der Aminosäuren Glutamin und Lysin (siehe Abb. 1). Durch diese irreversible Vernetzung von Aminosäuren bzw. Proteinen und Peptiden können hochmolekulare unlösliche Polymere erzeugt werden. Ein bekannter Vertreter der Transglutaminase-Familie, deren Mitglieder gemäß der IUPAC-Nomenklatur korrekterweise als "Protein-Glutamin: Amin-γ-Glutamyltransferasen" bezeichnet werden, ist der für die Blutgerinnung essentielle Faktor XIII. Nach Aktivierung von Faktor XIII durch Abspalten einer Pro-Sequenz (Pro-Peptid) kann das Enzym in Gegenwart von Calciumionen und unter Abspaltung von Untereinheiten an Fibrinfasern binden und diese miteinander vernetzen. So entsteht ein wasserunlöslicher Thrombus, der dem Wundverschluß dient.

Die biologischen Funktionen weiterer Mitglieder dieser Enzymfamilie sind vielfältig. So scheint beim Menschen ein kausaler Zusammenhang zwischen der Fischschuppenkrankheit (lchthyosis) und Mutationen in dem für die Keratinocyten-Transglutaminase kodierenden Gen zu bestehen (Huber et al., 1995, Science, 267, S. 525-528). Die lebensbedrohende Hauterkrankung unterstreicht die Bedeutung der Keratinocyten-Transglutaminase für den Aufbau des *Stratum corneum.* Diese kovalent vernetzte und verhornte Proteinschicht trägt maßgeblich zur Barrierefunktion der Haut bei (Candi et al., 1995, J. Biol. Chem. 44(3), S. 26382-26390).

Weiterhin gilt als gesichert, daß Gewebe-Transglutaminasen von Säugern am programmierten Zelltod (Apoptose) (Fesus et al., 1991, FEBS Lett. 284, S. 109-112; Zhang et al., 1995, J. Biol. Chem. 270, S. 6022-6029), am Aufbau und der Stabilisierung der extrazellulären Matrix (Aeschlimann und Paulsson, 1994, Thromb. Haemostasis 71, S. 402-415) sowie an der Heilung von Gewebeläsionen und Hautverletzungen beteiligt sind (Zatloukal et al., 1992, Lab. Invest. 66, S. 774-777; Bowness et al., 1988, Biochim. Biophys. Acta 967, S. 234-240). Die für die Transglutaminase-Aktivität charakteristische Bildung von hochmolekularen Proteinaggregaten trägt ebenfalls zur Alterung humaner Erythrocyten bei. Bedingt durch Fehlfunktionen von Ionenpumpen kommt es zu einer Erhöhung der intrazellulären Calciumkonzentration und damit zur Aktivierung der calciumabhängigen Transglutaminasen. Aus dem Aufbau kovalenter Brücken zwischen Membranproteinen und dem Cytoskelett resultiert eine irreversible Versteifung der Zellmembran. Die damit verbundenen morphologischen Veränderungen sind unmittelbar auf die Bildung von Isopeptidbindungen zurückzuführen (Lorand und Conrad, 1984, Mol. Cell. Biochem. 58, S. 9-35). Neben der Bildung von hochmolekularen Proteinaggregaten, der physiologischen Hauptfunktion von Transglutaminasen, wird ein weiteres Prinzip der posttranslationalen Modifizierung von Proteinen und Peptiden diskutiert. Cordella-Miele und Kollegen fanden beispielsweise, daß die katalytische Aktivität von Phospholipase A₂ sowohl durch den Einbau von Polyaminen, als auch durch die Verknüpfung zu einem Homodimer signifikant erhöht wird (Cordella-Miele et al., 1993, J. Biochem. 113, S. 164-173). Ähnlich dazu erlangt Interleukin-2 nach Dimerisierung durch eine Transglutaminase eine neue biologische Eigenschaft: dieses Cytokin besitzt lediglich in seiner dimeren Form eine Cytotoxizität gegen Oligodendrocyten (Eitan und Schwartz, 1993 Science 261, S. 106-108).

Während die verschiedenen Transglutaminase-Enzyme, die in Säugetieren identifiziert worden sind, Molekulargewichte von 65 kDa bis zu 320 kDa (Heterotetramer des Plasmafaktors XIII(a)) haben und Calciumionen als Kofaktor für ihre vollständige Aktivierung benötigen, zeichnen sich bakterielle Transglutaminasen durch relativ kleine Molekulargewichte (ca. 38-44 kDa) und eine calciumunabhängige Aktivität aus.

Den Transglutaminasen kommt eine große wirtschaftliche Bedeutung zu, da in der Pharma-, Kosmetik- und Lebensmittelindustrie vermehrt biologische Polymerisierungsmittel nachgefragt werden. Durch Vernetzung und Desamidierung werden in erster Linie die Eigenschaften der Produkte verbessert, z.B. Textur-, Gel- und Bruchfestigkeit, Viskosität und Elastizität oder Mineralstoffbindung. Zusätzlich lassen sich unangenehme Begleiterscheinungen von Herstellungsverfahren, wie Bitterkeit oder unangenehmer Geruch, beseitigen. Darüber hinaus könnten Transgtutaminasen zukünftig bei der Immobilisierung von Enzymen und Antikörpern, beispielsweise für Biosensoren oder Immundiagnostiktests oder zur Herstellung von Emulgatoren, eine wichtige Rolle spielen.

Bisherige Verfahren zum Vernetzen von Produkten auf Proteinbasis setzen jedoch überwiegend chemische oder thermische Behandlungsmethoden ein. Die Verwendung von chemischen Reagenzien ist dabei umstritten, da es sich in der Regel um bifunktionelle organische Moleküle handelt, die auch mit körpereigenen Proteinen reagieren, was sich in einer hohen Toxizität ausdrückt. Diese Verbindungen sind starke Reizstoffe der Atemwege, der Haut und der Augen (z.B. Formaldehyd, Glyoxal, Glutardialdehyd, Hexamethylendiisocyanat, Toluen-2,4-Diisocyanat, Diazobenzidin). Da für die Erzeugung menschlicher Lebensmittel keine toxischen Reagenzien eingesetzt werden sollten, wird in diesen Fällen oft ein thermisch induziertes Polymerisierungsverfahren eingesetzt. Nachteil dieser Verfahren ist jedoch, daß gleichzeitig essentielle Inhaltsstoffe, wie z.B. Vitamine, zerstört werden.

Als Alternative zu den oben erwähnten Verfahren hat sich der Einsatz von Transglutaminase-Enzymen, die aus Säugerzellen gereinigt wurden, nicht durchsetzen können, da deren Verwendung aufgrund der aufwendigen Proteinreinigung, der eingeschränkten Verfügbarkeit und mangelnden Lagerfähigkeit nicht rentabel ist.

Durch die Aufklärung einer Nukleinsäuresequenz einer bakteriellen Transglutaminase durch Washizu und Kollegen gelang es zum ersten Mal, rekombinante Transglutaminase mit dem Wirtsorganismus *Streptomyces lividans* in größeren Mengen zur Verfügung zu stellen (Washizu et al., 1994, Biosci. Biotech. Biochem. 58, S. 82-87 und EP 0 481 504). Takehana und Kollegen haben die rekombinante Herstellung der aktiven Form des bakteriellen Enzyms in E. coli beschrieben, wobei diese aktive Form für die Wirtszellen cytotoxisch ist (Takehana et al., 1994, Biosci. Biotech. Biochem. 58, S. 88-92). Die Expressionsausbeuten lagen in beiden Fällen noch weit unter der optimalen Ausbeute.

Es ist daher Aufgabe der vorliegenden Erfindung, ein bakterielles, gegebenenfalls gezielt aktivierbares Transglutaminase-Enzym sowie Mittel und Wege zu dessen Herstellung und Aktivierung zur Verfügung zu stellen. Die Aufgabe wird erfindungsgemäß gelöst durch ein Transglutaminase-Polypeptidmolekül gemäß der folgenden Formel:

R₁-R₂-R₃

wobei
R₁ entweder Wasserstoff, Methionin oder eine Aminosäure-Pre-Sequenz bedeutet;
R₂ eine Aminosäure-Pro-Sequenz von *Streptoverticillium mobaraense* bedeutet, wobei R₂ die folgende Sequenz hat: und
R₃ eine Aminosäure-Sequenz für ein aktives bakterielles Transglutaminase-Polypeptid aus *Streptoverticillium mobaraense* bedeutet, wobei R₃ die folgende Sequenz hat:

Die Aufgabe wird weiter gelöst durch ein Transglutaminase-Nukleinsäuremolekül gemäß der Formel:

N₁-N₂-N₃

wobei
N₁ eine 5'-Phosphatgruppe, eine 5'-OH-Gruppe oder eine Nukleinsäuresequenz bedeutet, die für Methionin oder eine Pre-Sequenz kodiert;
N₂ eine Nukleinsäuresequenz bedeutet, die für eine Pro-Sequenz aus *Streptoverticillium mobaraense* kodiert, wobei N₂ die folgende Sequenz umfasst: und
N₃ eine Nukleinsäuresequenz bedeutet, die für ein aktives bakterielles Transglutaminase-Polypeptidmolekül aus *Streptoverticillium mobaraense* kodiert, wobei N₃ die folgende Sequenz umfasst:

Unter einem "Homolog" eines Transglutaminase-Polypeptidmoleküls wird hier ein Polypeptid mit Transglutaminaseaktivität verstanden, das in seiner Sequenz eine Übereinstimmung von 60% oder mehr mit den erfindunsgemäßen Transglutaminase-Polypeptiden aufweist. Bevorzugt ist eine Übereinstimmung von 70% oder mehr und besonders bevorzugt ist eine Übereinstimmung von 80% oder mehr; insbesondere bevorzugt sind Transglutaminase-Polypeptide mit Homologen von 90% oder 95%.

Unter "stringenten Hybridisierungsbedingungen" werden Bedingungen verstanden, wie sie z.B. in Maniatis T., Sambrook, J. und Fritsch, E.F., 1989, Molecular Cloning, a Laboratory Manual, 2. Aufl., C.S.H. Laboratory Press, beschrieben sind.

Im erfindungsgemäßen Polypeptid R₁-R₂-R₃ dient die Pre-Sequenz R₁, die ebenfalls als Signalsequenz, Leitsequenz oder Prä-Sequenz bezeichnet wird, dazu, das Protein in ein bestimmtes Zellkompartiment zu leiten und dort in der Membran zu verankern. Pre-Sequenzen haben daher überwiegend hydrophoben Charakter. In Eukaryonten werden die Pre-Sequenzen dabei z.B. im Falle der in das endoplasmatische Retikulum (ER) eingeschleusten Pre-Proteine noch während der Synthese der restlichen Peptidkette auf der äußeren Seite der ER-Membran von spezifischen Signalpeptidasen im ER-Lumen abgespalten. In Prokaryonten dienen aminoterminale Signalsequenzen, die den eukaryontischen Sequenzen sehr ähnlich sind, dazu, die Bakterienproteine zur Plasma- und zur äußeren Membran zu dirigieren. Analog zu den Eukaryonten wird auch bei Prokaryonten die Pre-Sequenz durch eine Signalpeptidase abgespalten, um das Protein freizusetzen.

Pro-Sequenzen, die ebenfalls in Eu- und Prokaryonten auftreten, kodieren für Sequenzen, die das restliche C-terminal gelegene Polypeptidmolekül in einer inaktiven (unreifen) Konformation erhalten. Durch kontrollierte Abspaltung dieser Pro-Sequenz mittels hochspezifischer Peptidasen kann die Aktivierung der betreffenden Proteine reguliert werden. Die Blutgerinnungskaskade von Säugern ist ein bekanntes Beispiel für die sequenzielle Aktivierung mehrerer Pro-Enzyme durch enzymatische Abspaltung der jeweiligen Pro-Sequenz.

Kanaji et al. (J. Biol. Chem. 268, 11565-11572) publizierten 1993 die erste Proteinsequenz für eine mikrobielle Transglutaminase aus *Streptoverticillium S-8112.* Washizu et al. bestätigten 1994 diese Aminosäuresequenz und veröffentlichten die Nukleinsäuresequenz einer bakteriellen Transglutaminase (Washizu et al., 1994, Biosci. Biotech. Biochem. 58, 82-87 und EP 0 481 504). Die in dieser Veröffentlichung angegebene Pre- und Pro-Sequenz des Enzyms haben sich nicht verifizieren lassen. Seitdem sind keine weiteren Aminosäure- oder Nukleinsäuresequenzen bekannt geworden. In der vorliegenden Erfindung wird nunmehr die inaktive Form einer bereits bekannten Transglutaminase mit einer funktionellen Pro-Sequenz offenbart.

In einer bevorzugten Ausführungsform ist R₁ der oben genannten Formel eine prokaryontische Pre-Sequenz. Diese kann z. B. aus der Gruppe der Gram-negativen und -positiven, aeroben und anaeroben Stäbchen und Kokken oder anderen Bakteriengruppen ausgewählt sein. Dabei sind in der vorliegenden Erfindung besonders Pre-Sequenzen bevorzugt, die für effiziente Expression und Ausschleusung der betreffenden Polypeptidsequenz in *E. coli* geeignet sind. Beispielhaft für eine bakterielle Pre-Sequenz sei die aminoterminale Signalsequenz von β-Lactamase genannt. Weitere Pre-Sequenzen eukaryontischer und prokaryontischer Gene sind z. B. in Singer und Berg, Gene und Genome, Spektrum Akademischer Verlag, 1992, zu finden. Generell ist ebenfalls eine Ablagerung von inaktiven Pro-Transglutaminasemolekülen in Form von "inclusion bodies" möglich.

In einer bevorzugten Ausführungsform ist R₁ der oben genannten Formel eine Aminosäure-Pre-Sequenz aus Actinomyceten. Die Gruppe der Actionmyceten umfaßt viele Gram-positive Kurzstäbchen und hyphen- und mycelartige Bakterien, zu denen auch die Streptomyceten, z.B. *Streptomyces lividans,* zählen. Bevorzugt sind Pre-Sequenzen von *Streptoverticillium* und besonders bevorzugt von *Streptoverticillium mobaraense* oder von *Streptoverticillium fervens subsp. melrosporus*.

Von den Erfindern ist beobachtet worden, daß Pro-Enzyme auch in den folgenden *Streptoverticillium*-Arten produziert werden:
*Streptoverticillium netropsis, Streptoverticillium eurocidicum, Streptoverticillium rubrochloricum, Streptoverticillium cinnamoneum, subsp. cinnamoneum, Streptoverticillium baldacci*.

Wie in Beispiel 4 aufgezeigt, wurde mit Hilfe degenerierter Oligonukleotide, die von den ermittelten Aminosäuresequenzen von *Streptoverticillium mobaraense* abgeleitet worden waren, die genomische DNA analysiert, um die Nukleinsäuresequenzen des Transglutaminase-Enzyms zu ermitteln.

Die vorliegende Erfindung stellt daher ebenfalls die oben genannten Transglutaminase-Nukleinsäuremoleküle zur Verfügung.

In einer bevorzugten Ausführungsform kodiert N₁ der oben angegebenen Formel eine Pre-Sequenz aus Actinomyceten. Bevorzugte Sequenzen stammen aus *Streptoverticillium* und besonders bevorzugt aus *Streptoverticillium mobaraense* oder aus *Streptoverticillium fervens subsp. melrosporus*. In weiteren bevorzugten Ausführungsformen kodiert N₁ für Pre- Sequenzen aus: *Streptoverticillium netropsis, Streptoverticillium eurocidicum, Streptoverticillium rubrochloricum, Streptoverticillium cinnamoneum, subsp. cinnamoneum, Streptoverticillium baldacci*.

Die oben erwähnten Nukleinsäuremoleküle können in einen geeigneten Expressionsvektor kloniert werden. Dieser Vektor kann zur Expression in Prokaryonten und auch Eukaryonten eingesetzt werden. Der Vektor wird dann durch geeignete Verfahren, z.B. Elektroporation, Hitzeschockbehandlung, Lipofektin-unterstützte Verfahren etc., in einen geeigneten Wirtsorganismus eingeführt.

Ein bevorzugter Wirtsorganismus zur Expression einer der oben genannten Transglutaminase-Nukleinsäuremoleküle ist ein Prokaryont, wobei Gram-negative Organismen, insbesondere *Escherichia coli* und ausgewählte Gram-positive Organismen, wie z.B. *Streptomyces lividans*, besonders bevorzugte Wirtsorganismen sind.

In einer weiteren bevorzugten Ausführungsform der vorliegenden Erfindung exprimiert der Wirtsorganismus, der einen erfindungsgemäßen Expressionsvektor enthält keine Protease, die die Bindung zwischen R₂ und R₃ spalten kann. Dies kann durch Wahl des Wirtsorganismus, durch die Wahl besonderer Kulturbedingungen, durch Inhibitoren oder durch Einführen von Defekten in das Proteasegen erreicht werden. Durch Inaktivierung bzw. in Abwesenheit einer solchen Protease unterbleibt die Spaltung des Transglutaminase-Pro-Enzyms in Pro-Sequenz und aktives Enzym, wodurch u.a. die Ausbeute an inaktivem Pro-Enzym erhöht wird.

Die vorliegende Erfindung stellt weiterhin ein Verfahren zum Herstellen eines inaktiven Pro-Polypeptidmoleküls einer Transglutaminase wie oben beschrieben durch Kultivieren eines Wirtsorganismus zur Verfügung, wobei der in dem Wirtsorganismus enthaltene Expressionsvektor eine der oben beschriebenen Nukleinsäuren umfaßt, und weiterhin durch Ernten des Pro-Polypeptidmoleküls aus dem Kulturüberstand und/oder des Pre-Pro- oder Pro-Polypeptidmoleküls aus den Wirtszellen. Dabei ist die Herstellung eines inaktiven Transglutaminaseenzyms aus Actinomyceten, insbesondere aus *Streptoverticillium* und besonders aus *Streptoverticillium mobaraense* und *Streptoverticillium fervens subsp. melrosporus* bevorzugt. Diese Enzyme können von einigen Stunden bis mehreren Tagen nach Expressionsbeginn aus dem Kulturüberstand geerntet werden,
wobei der überwiegende Teil des Proteins als Pro-Enzym vorliegt und gegebenenfalls ein geringer Teil durch im Kulturmedium befindliche Proteasen bereits zum aktiven Enzym gespalten wird.

Erfindungsgemäß kann das Transglutaminase-Pro-Polypeptidmolekül durch chemische, physikalische oder enzymatische Abspaltung der R₂-Sequenz (oder R₁-R₂-Sequenzen) von R₃ aktiviert werden.

Besonders bevorzugt ist dabei eine enzymatische Spaltung, die mit Hilfe von Dispase, Trypsin oder Chymotrypsin durchgeführt wird. Eine sequenzspezifische Spaltung ist z.B. mit den Reagenzien Bromcyan (Met) und Hydroxylamin (Asn-Gly) zu erzielen. Weiterhin können geringe Änderungen der folgenden Parameter die Spaltung eines Proteins beeinflussen: pH-Wert, Ionenstärke und -zusammensetzung, Temperatur, Substratkonzentration, chaotrope Agenzien oder Glycerin, z.B. 10% Essigsäure mit Pyridin auf pH 2,5 eingestellt (in An- oder Abwesenheit von 7M Guanidiniumchlorid; 70% Ameisensäure (G. Allen, Sequencing of proteins and peptides, 2. Aufl. 1989, S. 79-82, Elsevier Amsterdam).

Weiterhin bezieht sich die Erfindung auf ein Verfahren zum kovalenten Verknüpfen von Proteinen und/oder Peptiden unter Verwendung eines erfindungsgemäßen Transglutaminase-Pro-Polypeptidmoleküls, wobei die Proteine und/oder Peptide unter geeigneten Versuchsbedingungen zunächst mit dem Transglutaminase-Pro-Polypeptidmolekül in Kontakt gebracht werden. Die Transglutaminase kann dann zu einem gewählten Zeitpunkt durch Spaltung aktiviert werden, wodurch die Verknüpfungsreaktion gestartet wird.

Die folgenden Abbildungen und Beispiele erläutern die Erfindung.

### Beschreibung der Abbildungen

### Abbildung 1:

Reaktionsschema von Transglutaminase-Enzymen. Die γ-Carboxamidgruppe von Protein-gebundenem Glutamin wird unter Freisetzung von Ammoniak auf ein primäres Amin übertragen. Wenn als Glutamylakzeptor die ε-Aminofunktion eines ebenfalls Proteingebundenen Lysins fungiert, resultiert entsprechend eine inter- bzw. intramolekulare Isopeptidbindung, abhängig davon, ob eine zweite oder dieselbe Peptidkette als Amindonor beteiligt ist.

### Abbildung 2:

Kultivierungsverlauf von *Streptoverticillium fervens subsp. melrosporus*.
A) Aktivitätstest zur Bestimmung der Transglutaminaseaktivität im Kulturüberstand.
B) Western-Blot des Flüssig-Kulturmedium mit anti-Transglutaminase-Antiserum. Die Spuren 1-7 entsprechen den Zeitpunkten der unter 2A) dargestellten Aktivitätsbestimmungen nach 24, 43, 48, 64, 70, 90 und 95 h.

### Abbildung 3:

Reinigung der bakteriellen Transglutaminase von *Streptoverticillium mobaraense:*
A) Typisches Elutionsprofil einer Kationenaustausch-Chromatographie. Die Transglutaminase enthaltenden Fraktionen sind schwarz unterlegt.
B) Silbergefärbtes SDS-Polyacrylamid-Gel der Transglutaminase-Fraktionen.
Spur M: Markerproteine: 66 kDa, 45 kDa, 29 kDa, 20 kDa, 14 kDa; Spuren 1-6: Fraktionen, die zwischen 197-202 min bei der Chromatographie eluierten.

### Abbildung 4:

A) Reinigung der bakteriellen Transglutaminase von *Streptoverticillium fervens subsp. melrosporus:* Elutionsprofil einer Kationenaustausch-Chromatographie bei pH 6,0 (die Enzym-Fraktionen sind unterlegt).
B) Rechromatographie der teilgereinigten Transglutaminase bei pH 5,0 (die Enzym-Fraktionen sind unterlegt).
C) Silbergefärbtes SDS-Polyacrylamid-Gel der Transglutaminase-Fraktionen. Spuren 1-5: Fraktionen, die zwischen 80-85 min bei der Rechromatographie eluierten.

### Abbildung 5:

Reinigung der bakteriellen Pro-Transglutaminasen von *Streptoverticillium fervens subsp. melrosporus* und *Streptoverticillium mobaraense* durch Kationenaustausch-Chromatographie bei pH 5,0
A) Elutionsprofil der Reinigung des Pro-Enzyms von *Streptoverticillium mobaraense*. Die Pro-Transglutaminase eluiert bei einer Salzkonzentration von 0,4 M , die aktive Transglutaminase bei 0,5 M NaCl. Die entsprechenden Fraktionen sind schwarz unterlegt. Der Nachweis der Proteine erfolgte mittels Western-Blot-Analyse.
B) Elutionsprofil der gefällten Proteine von *Streptoverticillium fervens subsp. melrosporus.* Die Pro-Transglutaminase wird bei einer Salzkonzentration von 0,28 M, die aktive Transglutaminase bei 0,35 M NaCl eluiert.

### Abbildung 6:

Strategie zur Bestimmung von kodierenden Nukleinsäuresequenzen der bakteriellen Pro-Transglutaminase von *Streptoverticillium fervens subsp. melrosporus* und *Streptoverticillium mobaraense*.

Aus den für beide Enzyme ermittelten Aminosäuresequenzen der Pro-Enzyme (schwarz unterlegt) wurden Oligonukleotide abgeleitet, und mit Hilfe des Oligonukleotids RP1224AS (siehe Tabelle 6) wurde der gesamte ca. 1,1 kBp umfassende DNA-Bereich mittels PCR amplifiziert. Anschließend erfolgte, wie duch die Pfeile schematisch dargestellt, die überlappende Sequenzierung des kodierenden Strangs sowie des Gegenstrangs.

### Abbildung 7:

Proteolytische Aktivierung der Pro-Transglutaminase von *Streptoverticillium mobaraense* mit Dispase. Je 20 µg des Pro-Enzyms wurden in einer seriellen Verdünnungsreihe von 10 µg (Spur 6), 5 µg (5), 2,5 µg (4), 1,25 µg (3), 0,625 µg (2), 0,3125 µg (1) Dispase für die Dauer von 30 min bei einer Temperatur von 37°C inkubiert und mit Hilfe einer SDS-PAGE aufgetrennt.

Die Proteine des Gels wurden auf einen Filter übertragen, der mit einem Antikörper für bakterielle Transglutaminase inkubiert wurde.

### Abbildung 8:

Vergleich der Thermostabilität der Pro-Transglutaminase (schwarze Kreise) und des aktiven Enzyms (weiße Kreise) von *Streptoverticillium mobaraense*.

Beide gereinigten Proteine wurden für 60 min bei den angegebenen Temperaturen inkubiert und dann auf 37°C temperiert. Nach Aktivierung des Pro-Enzyms mit Dispase wurde die Transglutaminase-Aktivität bestimmt. Die Angaben sind Relativwerte und beziehen sich jeweils auf die Probe, die bei 18°C (Raumtemperatur) inkubiert wurde.

### Abbildung 9:

Analyse der Spezifität des Antiserums (Verdünnung 1:20.000) gegen bakterielle Transglutaminase mittels Western-Blot.
Spur 1: Kulturmedium (*Streptoverticillium mobaraense*)
Spur 2: Zur Immunisierung eingesetztes gereinigtes Transglutaminase-Protein

### 1. Kultivierung von Streptoverticillien

### 1a) Plattenkulturen zum Inokulieren von Flüssigmedien und zur Stammkonservierung

Die Anzucht der Mikroorganismen zur Inokulation von Flüssigmedien sowie das Anlegen von Dauerkulturen erfolgte auf GYM-Agarplatten (Shirling und Gottlieb, 1966, Int. J. Syst. Bacteriol. 16, S. 313-340). Ausgehend von einer Reinkultur der DSMZ (Deutsche Sammlung von Mikroorganismen und Zellkulturen GmbH) wurden die Bakterienstämme *Streptoverticillium mobaraense* und *Streptoverticillium fervens subsp. melrosporus* in sterilem Wasser aufgenommen. Die Suspension wurde mit einer Pipette auf die GYM-Platten überführt und mit einem Drigalski-Spatel verteilt.

Vor der Inokulation von Flüssigkulturen wurden Vorkulturen auf einer neuen Agarplatte angelegt. Hierzu wurden Bakterienkolonien mit einer Impföse auf der Platte ausgestrichen und bei 28 - 30°C mindestens 5 Tage inkubiert, bis neben dem Substratmycel auch sporulierende Lufthyphen zu sehen waren.

Zur Stammkonservierung wurden die Streptoverticillien nach etwa 20 Tagen auf eine neue GYM-Agarplatte überimpft. Die Petrischalen wurden anschließend mit einem Kunststoffilm verschlossen und bei 28-30°C inkubiert.

### 1b) Flüssigkulturen zur präparativen Herstellung von bakteriellen Transglutaminasen

Zur präparativen Enzymproduktion von bakteriellen Transglutaminasen wurden 1 l Erlenmeyer-Kulturkolben verwendet, die mit 110 ml Flüssig-Medium gefüllt wurden. Nach dem Autoklavieren der Kulturmedien wurde über einen Sterilfilter 1 ml Spurenelementlösung zugegeben und mit einer Vorkultur, die auf GYM-Agarplatten angezüchtet wurde, beimpft.

Die Kultivierung erfolgte im Brutschrank bei etwa 29°C bis zum Erreichen des gewünschten Transglutaminase-Produkts. Der Sauerstoffeintrag, der für das Wachstum der aeroben Bakterien benötigt wird, erfolgte durch intensive Bewegung der Flüssigkultur auf einem Querschüttler mit einer Frequenz von 103 pro Minute. Nach zwei bis vier Tagen wurde das Produkt-enthaltende Kulturmedium von den Mikroorganismen durch Zentrifugation und Filtration weitgehend getrennt und direkt weiter verarbeitet oder bei - 20°C gelagert.

### 1c) Kultivierung von Streptoverticillium mobaraense

Die Anzucht und Stammkonservierung der Mikroorganismen erfolgte auf Agarplatten, ausgehend von einer Reinkultur der DSMZ. Die Submerskultivierung zur Transglutaminaseproduktion erfolgte, wie unter 1 b) beschrieben, in einem Komplex-Medium, das zusätzlich mit Spurenelementen angereichert war (Voelskow, 19, Jahrbuch Biotechnologie, Carl Hanser Verlag, München, S. 343-361). Nach einer Kultivierungsdauer bis zu zwei Tagen für das Pro-Enzym und von drei bis maximal vier Tagen für reife Transglutaminase-Polypeptidmoleküle wurden die Bakterien durch Zentrifugation und Sterilfiltration vom Flüssigmedium, in das die Mikroorganismen das Produkt sekretieren, getrennt. Die volumenbezogene Aktivität für reife Transglutaminase lag mit 3,0 - 3,5 E/ml deutlich über den publizierten Werten (2,5 E/ml in Ando et al., 1989, Agric. Biol. Chem. 53, S. 2613-2617; 1,5-2,0 E/ml in Gerber et al., 1994, Biochem. J. 299, S. 825-829), was offensichtlich auf die veränderten Kultivierungsbedingungen zurückzuführen war. Die Menge der reifen Transglutaminase lag durchschnittlich bei mehr als 80 mg Enzym pro Liter Kulturmedium. Die unter 1 a bzw. 1 b beschriebenen Fermentationsbedingungen ermöglichen somit eine außerordentlich effiziente Produktion der bakteriellen Transglutaminase.

### 1d) Kultivierungsverlauf von Streptoverticillium fervens subsp. melrosporus

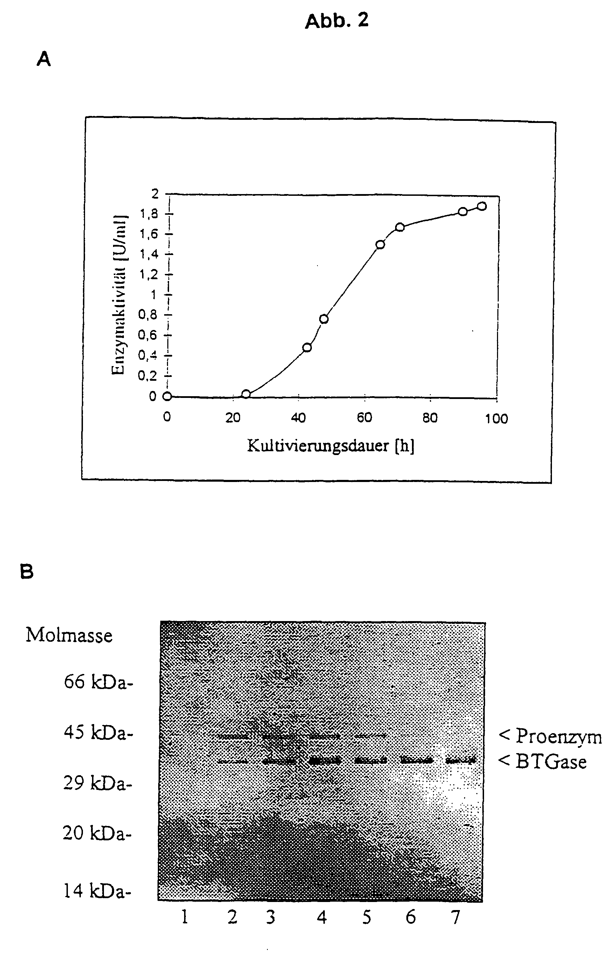

Die Anzucht und die Kultivierung des Stammes entsprachen den für die Reinigung der bakteriellen Transglutaminase von *Streptoverticillium mobaraense* optimierten Bedingungen (siehe 1 c)). Die Fermentation wurde parallel durch die Bestimmung der Transglutaminaseaktivität und mittels Western-Blot-Analyse verfolgt. Die Ergebnisse sind in Abb. 2 dargestellt.

### 2. Bestimmung der Enzymaktivität von Transglutaminasen

Die eingesetzten Aktivitätstests für Transglutaminasen beruhen auf der Umsetzung eines Modellsubstrats unter definierten Bedingungen zu einem bekannten Produkt. Abhängig vom Testsystem konnte die enzymatische Aktivität quantifiziert oder lediglich eine qualitative Aussage getroffen werden. Bei allen eingesetzten Nachweisen handelt es sich um nicht kontinuierliche Bestimmungsmethoden, bei denen die Reaktion zu einem bestimmten Zeitpunkt abgebrochen wurde. Bei den quantifizierbaren Aktivitätsbestimmungen wurde die Produktkonzentration direkt oder nach Abtrennen der Edukte bestimmt. Als Glutamyldonorsubstrate wurden entweder synthetische Dipeptidderivate oder das Milchprotein Casein verwendet (Pasternack, 1998, Dissertation, Technische Universität Darmstadt).

### 3. Säulenchromatographische Methoden zur Reinigung von bakteriellen Transglutaminasen und ihrer Pro-Enzyme

Ausgangsmaterial zur chromatographischen Reinigung von mikrobiellen Transglutaminasen und ihrer Pro-Enzyme war das zellfreie, durch Ethanolfällung oder Druckfiltration eingeengte Flüssigkulturmedium.

Die vollständige Reinigung der Enzyme und ihrer inaktiven Vorstufen erfolgte mittels einer Niederdruck-LC-Anlage an einem stark sauren Kationenaustauschermaterial in Anlehnung an das Verfahren von Gerber und Kollegen (Gerber et al., 1994, Biochem. J., 299, S. 825-829). Die Reinigung wurde dann, wenn nötig, durch eine zusätzliche Rechromatographie von teilgereinigten Fraktionen vervollständigt. Die Verwendung eines Puffers mit einem niedrigeren pH-Wert ermöglichte hierbei eine stärkere Bindung der Proteine an den Ionenaustauscher. Bei ansonsten gleichen Chromatographiebedingungen konnte dadurch eine weitergehende Reinigung der Transglutaminasen und ihrer Pro-Enzyme erzielt werden.

Das konzentrierte Flüssigkulturmedium wurde zum Abtrennen größerer Partikel zentrifugiert (10000 x g, 4°C, 10 min) und die Lösung durch Sterilfiltration von Mikroorganismen und Schwebstoffen befreit. Die Transglutaminasen wurden durch Kationenaustausch-Chromatographie, entsprechend nachstehender Übersicht, aus der Kulturbrühe gereinigt bzw. angereichert. Alle Solventien wurden mit bidestilliertem Wasser angesetzt, vor Gebrauch sterilfiltriert und im Ultraschallbad entgast.

**Tabelle 1: Elutionsbedingungen für bakterielle Transglutaminasen**

| | **Puffer A** | **Puffer B** | |
|---|---|---|---|
| **Funktion** | (Standardchromatographie) (%) Puffer C (Rechromatographie) | (Standardchromatographie) (%) Puffer D (Rechromatographie) | **Volumen** (ml) |
| Auftrag der Proteinlösung | - | - | max. 90 |
| Elution der nichtbindenden Proteine | 100 | 0 | ca. 195 |
| Linearer Gradient (I) | 100-90 | 0-10 | 195 |
| Plateau | 90 | 10 | ca. 195 |
| Linearer Gradient (II) | 90-70 | 10-30 | 195 |
| Linearer Gradient (III) | 70-0 | 30-100 | 455 |

Die Chromatographie erfolgte bei Raumtemperatur. Alle Protein enthaltenden Fraktionen wurden durch Enzymaktivitätstests, SDS-PAGE, Western-Blots und Proteinbestimmungen charakterisiert. Die Transglutaminase enthaltenden Fraktionen wurden gegebenenfalls vereinigt und bei -20°C gelagert.

Vor der Durchführung einer Rechromatographie wurden die entsprechenden Fraktionen entsalzt und auf den Acetatpuffer eingestellt. Nach Sterilfiltration wurde die Proteinlösung auf die entsprechend äquilibrierte Säule aufgetragen. In der Regel wurden die Proteine bei einer Salzkonzentration, die 0,15 bis 0,25 M höher als bei der Standardchromatographie lag, eluiert.

| | | |
|---|---|---|
| Geräte | Niederdruck-LC-Anlage Merck/Hitachi | |
| Stationäre Phase | Fractogel EMD SO₃ 650 (S) | |
| | Durchmesser 2,6 | |
| | Betthöhe 13,5 cm | |
| | Bettvolumen 69 cm³ | |
| Mobile Phasen | Standardchromatographie | |
| | 50 mM Natriumphoshatpuffer pH 6,0 | (Puffer A) |
| | 1 M Natriumchlorid in Phosphatpuffer pH 6,0 | (Puffer B) |
| | Rechromatographie | |
| | 50 mM Natriumacetatpuffer pH 5,0 | (Puffer C) |
| | 1 M Natriumchlorid in Acetatpuffer pH 5,0 | (Puffer D) |
| Flußrate | 6,5 ml/min | |
| Injiziertes Volumen | max. 90 ml | |
| Detektion | Kontinuierliche Messung der Absorption bei λ =280 nm | |

### 3a. Reinigung der Transglutaminase von Streptoverticillium mobaraense

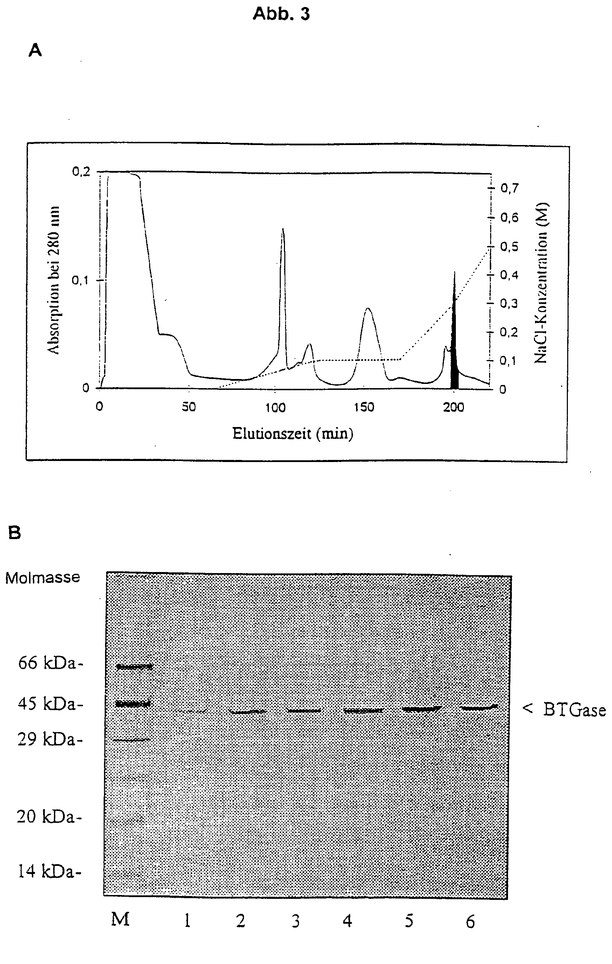

Vor der chromatographischen Reinigung wurde das Protein enthaltende Kulturmedium durch Ultrafiltration oder Ethanolfällung konzentriert bzw. umgepuffert, um die Trennleistung der Kationenaustausch-Chromatographie zu verbessern. Wie aus den nachfolgend dargestellten Elutionsprofilen hervorgeht, bindet eine Vielzahl der Proteine unter den angegebenen Chromatographie-Bedingungen nicht an das Säulenmaterial. Die Elution schwach gebundener Proteine erfolgte durch einen linearen Salzgradienten. Die Konzentration von 0,1 M Natriumchlorid wurde zusätzlich für die Dauer von etwa 60 min beibehalten. Im Anschluß wurde die bakterielle Transglutaminase durch einen zweiten, steileren Gradienten bei einer Salzkonzentration von 0,3 M Natriumchlorid eluiert (Abb. 3).

Das Polyacrylamid-Gel belegt die vollständige Reinigung der bakteriellen Transglutaminase von *Streptoverticillium mobarense* (Abb. 3A). Die anhand der Markerproteine bestimmte Molmasse von ca. 38 kDa stimmt mit den Literaturdaten überein. In Tabelle 2 sind die Reinigungsergebnisse zusammengefaßt:

**Tabelle 2: Typische Reinigungstabelle einer Transglutaminase von Streptoverticillium mobaraense**

| | Volumen | Gesamtaktivität | Gesamtprotein | Spezifische Aktivität | Ausbeute | Reinigungsfaktor |
|---|---|---|---|---|---|---|
| Kulturmedium | 211 ml | 696 E | - | - | - | - |
| konzentriertes Kulturmedium | 51 ml | 610 E | 287 mg | 2,1 E/mg | 88% | - |
| gekennzeichnete Fraktionen | 52 ml | 560 E | 17,6 mg | 31,7 E/mg | 81% | 15 |
| Spitzenfraktionen (200-202) | 19,5 ml | 412 E | 10,4 mg | 39,6 E/mg | 59% | 19 |

Die ermittelte spezifische Aktivität des Enzyms in den Spitzenfraktionen von annähernd 40 E/mg liegt deutlich über den bisher publizierten Werten von 22,6 E/mg (Ando et al., 1989, Agric. Biol. Chem. 53, S. 2613-2617) bzw. 10 E/mg (Gerber et al., 1994, Biochem. J. 299, S. 825-829). Die Ausbeute von 80% in den schwarz unterlegten Fraktionen unterstreicht zusätzlich die Leistungsfähigkeit des Reinigungsverfahrens.

### 3b. Reinigung einer Transglutaminase von Streptoverticillium fervens subsp. melrosporus

In Anlehnung an das für die bakterielle Transglutaminase von *Streptoverticillium mobaraense* (siehe 3a) etablierte Reinigungsschema, wurde die Aufarbeitung des Flüssig-Kulturmediums von *Streptoverticillium fervens subsp. melrosporus* sowie die chromatographische Trennung der Proteine weitgehend beibehalten.

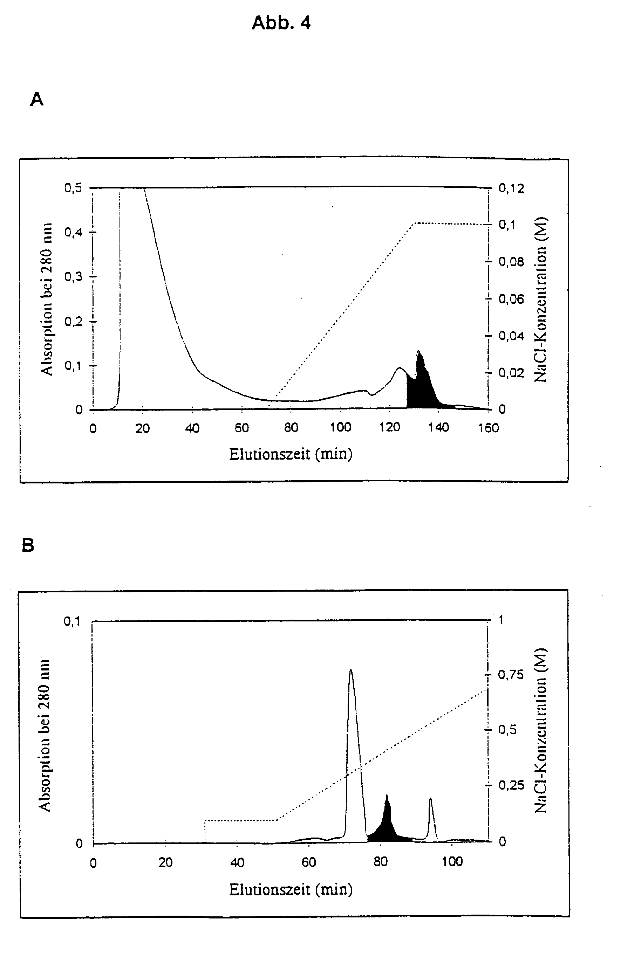

Im Gegensatz zu dem Transglutaminase-Enzym von *Streptoverticillium mobaraense* eluiert das Enzym von *Streptoverticillium fervens subsp. melrosporus* bereits bei einer Salzkonzentration von 0,1 M NaCl. Die Transglutaminase-Fraktionen verteilen sich zudem, wie aus dem Chromatogramm (Abb. 4a) ersichtlich ist, auf ein großes Elutionsvolumen.

Das Enzym liegt nach diesem Reinigungsschritt nicht rein vor, sondern ist noch mit Fremdproteinen kontaminiert. Zur weiteren Reinigung der Transglutaminase erwies es sich als sinnvoll, die in Abbildung 4a) gekennzeichneten Fraktionen zu vereinigen und die Proteine durch Rechromatographie zu trennen. Dabei wurde der pH-Wert um eine Einheit herabgesetzt, um eine stärkere Bindung an den Ionenaustauscher zu erreichen. In Abbildung 4b) ist ein typisches Elutionsprofil wiedergegeben.

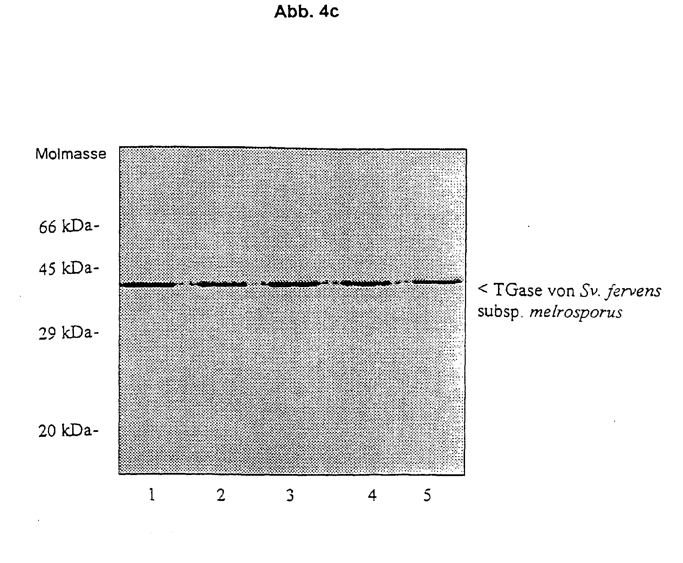

Die Überprüfung durch ein silbergefärbtes Polyacrylamid-Gel (Abb. 4c) belegt die vollständige Reinigung der neuen bakteriellen Transglutaminase von *Streptoverticillium fervens subsp. melrosporus*. Die anhand von Markerproteinen bestimmte Molmasse von ca. 38 kDa entspricht der Molmasse des Enzyms von *Streptoverticillium mobaraense*.

In Tabelle 3 sind die Ergebnisse des Reinigungsschemas für die neue bakterielle Transglutaminase von *Streptoverticillium fervens subsp. melrosporus*, ausgehend vom Flüssig-Kulturmedium, zusammengefaßt.

**Tabelle 3: Reinigungstabelle der Transglutaminase von Streptoverticillium fervens subsp. melrosporus**

| | Volumen | Gesamtaktivität | Gesamtprotein | Spezifische Aktivität | Ausbeute | Reinigungsfaktor |
|---|---|---|---|---|---|---|
| Kulturmedium | 215 ml | 202 E | - | - | - | - |
| konzentriertes Kulturmedium | 60 ml | 172 E | 593 mg | 0,29 E/mg | 85% | - |
| gekennzeichnete Chromatographie-Fraktionen | 104 ml | 141 E | 28,2 mg | 5 E/mg | 70% | 17 |
| gekennzeichnete Rechromatographie Fraktionen | 84,5 ml | 110 E | 6,2 mg | 17,8 E/mg | 54% | 61 |

### 3c. Reinigung bakterieller Pro-Transglutaminasen

Die Anzucht und Kultivierung der Mikroorganismen erfolgte, wie unter 1 c) beschrieben. Die Fermentation wurde bei der maximalen Konzentration der Pro-Enzyme beendet.

Die mit Ethanol präzipitierten Proteine wurden in Chromatographie-Puffer aufgenommen und unmittelbar danach auf den lonentauscher aufgetragen. Die proteolytische Aktivierung konnte so weitgehend verhindert werden.

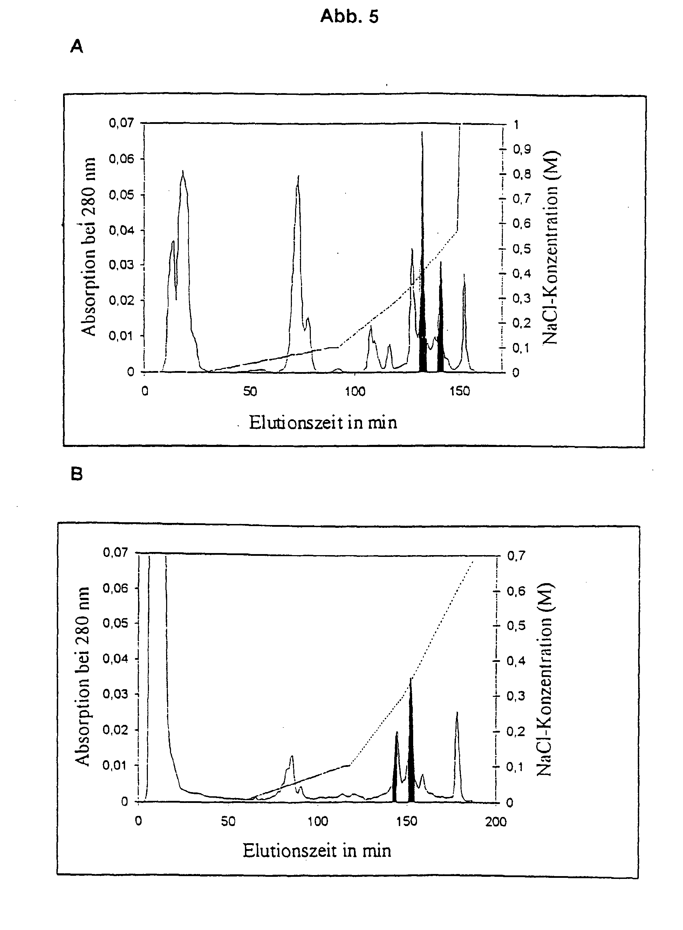

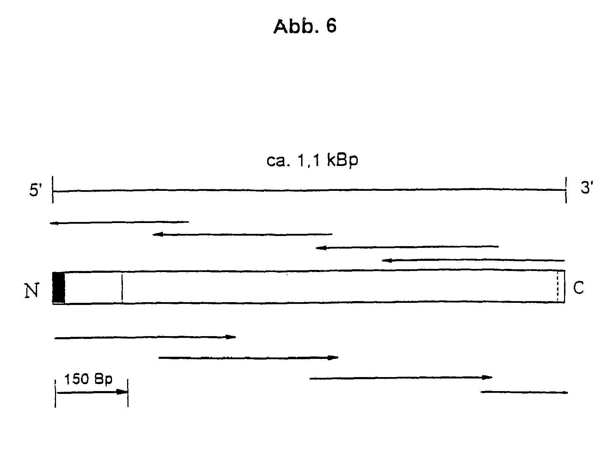

Die chromatographische Reinigung wurde bei pH 5,0 durchgeführt, um die Bindung der Pro-Enzyme, deren isoelektrische Punkte deutlich niedriger als die der aktiven Enzyme sind, an den Ionenaustauscher zu gewährleisten. Die Chromatogramme zur Isolierung der gefällten Proteine sind in Abbildung 5 wiedergegeben.

Mehrere proteinchemische Daten der gereinigten Pro-Enzyme sind vergleichend in Tabelle 4 gegenübergestellt.

**Tabelle 4: Vergleichende Gegenüberstellung proteinchemischer Daten der bakteriellen Pro-Transglutaminasen von Streptoverticillium fervens subsp. melrosporus und Streptoverticillium mobaraense.**

| Eigenschaften | Pro-Transglutaminase (*Streptoverticillium mobaraense*) | Pro-Transglutaminase (*Streptoverticillium fervens subsp. melrosporus*) |
|---|---|---|
| Molmasse (SDA-PAGE/Western-Blot) | ca. 45 kDa | ca. 45 kDa |
| Isolelektrischer Punkt (nach IEF) | 6,6 (aktives Enzym 8,0) | 6,1 (aktives Enzym 7,8) |
| Kultivierungsdauer bis zur maximalen Pro-Enzym-Konzentration (nach Western-Blot) | ca. 48 h | ca. 46 h |
| Salzkonzentration, bei der das Pro-Enzym eluiert (pH 5,0) | 0,4 M NaCl (aktives Enzym 0,5 M NaCl) | 0,28 M NaCl (aktives Enzym 0,35 NaCl) |
| Ausbeute des gereinigten Pro-Enzyms pro 100 ml Kulturmedium (Abb. 5, unterlegte Fraktionen) | 5,2 mg | 0,6 mg |

Von den gereinigten Pro-Enzymen wurden die N-terminalen Aminosäuresequenzen bestimmt. Bei Vergleich mit der von Washizu et al., 1994, Biosci. Biotech. Biochem. 58, S. 82-87 publizierten Primärstruktur, die auf der Nukleinsäuresequenz einer von den Autoren postulierten Pre-Pro-Region des Stamms *Streptoverticillium* S-8112 basiert, kann keine Homologie festgestellt werden.

### 4. Isolierung von qenomischer DNA aus Streptoverticillium

Die Präparation von genomischer DNA der Streptomyceten erfolgte mit Hilfe des QIAamp Tissue Kits von Quiagen gemäß den Herstellerhinweisen. Die Bakterienzellen wurden sedimentiert, die Hydrolyse des Mureins der Gram-positiven Mikroorganismen erfolgte im Anschluß durch Lysozym. Nach proteolytischem Abbau der intrazellulären Proteine wurde die DNA durch Bindung an eine Silicagel-Säule gereinigt.

Die Quantifizierung und Charakterisierung der isolierten Nukleinsäuren erfolgte spektralphotometrisch durch die Aufnahme eines kontinuierlichen UV-Spektrums zwischen 220 und 320 nm sowie durch Agarose-Gelelektrophorese. In Tabelle 5 sind die Ergebnisse zusammengefaßt.

**Tabelle 5: Ergebnisse der Isolierung von genomischer DNA aus Streptomyceten.**

| | *Streptoverticillium mobaraense* | *Streptoverticillium fervens subsp. melrosporus* |
|---|---|---|
| Volumen der präparierten DNA-Lösung | 180 µl | 200 µl |
| Extinktion der Nukleinsäurelösung bei 260 nm (Verdünnung 1:50) | 0,228 | 0,335 |
| Ausbeute an DNA | 103 µg | 168 µg |
| E_{260/280} | 2,0 | 1,87 |

### 5. Amplifizierung und Sequenzierung der kodierenden Genabschnitte der Pro-Transglutaminasen von Streptoverticillium fervens subsp. melrosporus und Streptoverticillium mobaraense

Die Nukleinsäuresequenzen der bakteriellen Pro-Transglutaminasen von *Streptoverticillium fervens subsp. melrosporus* und *Streptoverticillium mobaraense* wurde durch das PCR-Verfahren und anschließendes Sequenzieren der amplifizierten Nukleinsäuremoleküle aufgeklärt. Die verwendeten 5'-Oligonukleotide (s. Tabelle 6) beruhen auf den zuvor durch Edman-Abbau der gereinigten Pro-Enzyme ermittelten Aminosäuresequenzen des N-Terminus.

Als entsprechendes 3'-Oligonukleotid wurde die C-terminale Nukleinsäuresequenz einschließlich des TGA-Translationsterminations-Basentripletts von *Streptoverticillium* S-8112 übernommen und das Oligonukleotid (RP1224AS; Tabelle 6) erfolgreich zur Amplifizierung und Sequenzierung der beiden kodierenden Genabschnitte eingesetzt.

**Tabelle 6: Oligonukleotide, die zur Amplifizierung der für bakterielle Transglutaminasen kodierenden DNA-Abschnitte verwendet wurden**

| Bezeichnung der Oligonukleotide | Position | Aminosäuresequenz | Sequenz der Oligonukleotide |
|---|---|---|---|
| RP5'Mobaraense | - | DNGAGE | 5'GAC AAT GGC GCG GGG GAA3' |
| RP5'Fervens | - | AGSSDG | 5'GCC GGC AGC AGC GAC GGG3' |
| RP1224AS | 1241- 1224 | KQGWP(STOP) | 5'TCA CGG CCA GCC CTG CTT3' |

### Bedingungen für die PCR-Reaktion:

| Zeit (min) | Temperatur | Anzahl der Zyklen | Funktion |
|---|---|---|---|
| 2,0 | 94°C | | Denaturierung der DNA |
| | | | |
| 0,5 | 94°C | 10 | Denaturierung der DNA |
| 1,00 | von 65 auf 55°C in 1 °C Schritten ("touch-down") | | Hybridisierung (annealing) der Oligonukleotide |
| 1,5 | 72°C | | Amplifizierung der DNA |
| | | | |
| 0,5 | 94°C | 25 | Denaturierung der DNA |
| 1,0 | 55°C | | Hybridisierung der Oligonukleotide |
| | | | |
| 1,5 | 72°C | | Amplifizierung der DNA |
| | | | |
| 5,0 | 72°C | | Abschließende Amplifizierung |
| | | | |
| | 4°C | | Beenden der Reaktion |

Eine Probe jedes Ansatzes wurde entnommen und zur Überprüfung der Amplifizierung und zur Abschätzung der Länge der PCR-Produkte auf ein Agarosegel aufgetragen.

### 6. Proteolytische Aktivierung von Pro-Transglutaminase durch Dispase

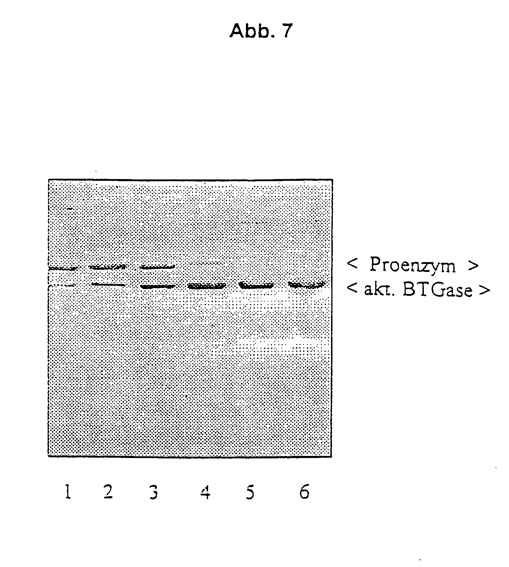

Es wurde gezeigt, daß die von *Streptoverticillium mobaraense* gereinigte Pro-Transglutaminase durch die Protease Dispase von *Bacillus polymyxa* aktiviert werden kann (Abb. 7).

Die Dispase hydrolysiert die Pro-Transglutaminase, wobei ein aktives Enzym mit einer Molmasse von etwa 38 kDa entsteht. Die aktive Transglutaminase ist unter den angegebenen Bedingungen resistent gegen weiteren Abbau durch die Protease. Darüber hinaus ist diese Prozessierung auch mit weiteren Proteasen möglich.

### 7. Vergleich der Thermostabilität von aktivem Transglutaminase-Enzym und Pro-Enzym

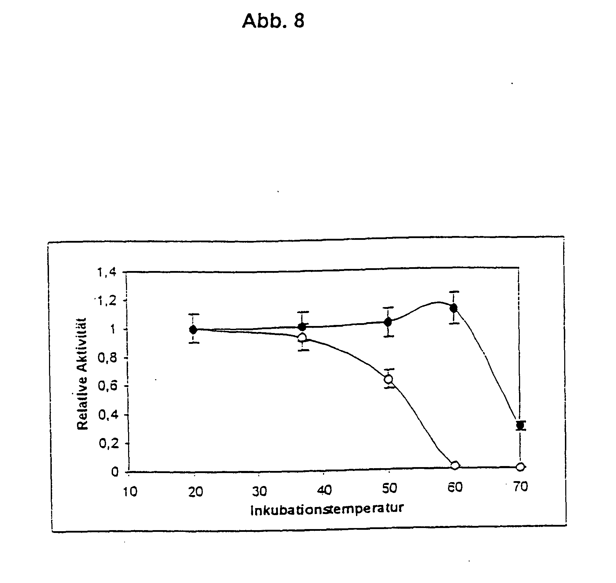

Die Grafik in Abbildung 8 belegt den Einfluß der Pro-Peptidsequenz auf die Stabilität des Pro-Enzyms. Bei Inkubation für die Dauer von 1 h bei 60°C und anschließender Aktivierung mit Dispase war kein Verlust der Transglutaminaseaktivität nachweisbar. Das reife Enzym verliert hingegen vollständig seine katalytische Aktivität. Selbst nach Inkubation bei 70°C ist bei der Pro-Transglutaminase noch ca. 30% der ursprünglichen Aktivität vorhanden.

### 8. Polyklonaler Antikörper gegen bakterielle Transglutaminase

Die zur Homogenität gereinigte Transglutaminase von *Streptoverticillium mobaraense* wurde zur Immunisierung eines Kaninchens eingesetzt. Die Spezifität der im Verlauf der Immunisierung entnommenen Probeentzüge sowie des Pre-Immunserums wurden über Western-Blot-Analyse mit dem heterogenen Kulturmedium und mit dem zur Immunisierung verwendeten Antigen überprüft.

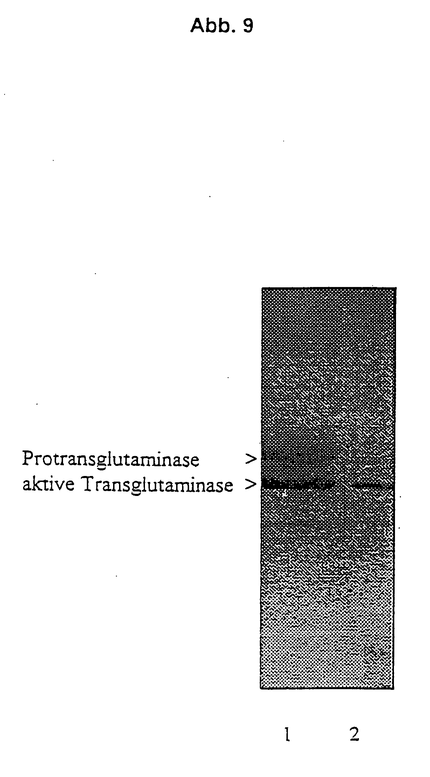

Der Western-Blot verdeutlicht, daß die erhaltenen Antikörper gegen die zur Immunisierung verwendete bakterielle Transglutaminase gerichtet sind (Abb. 9, Spur 2). Im Kulturmedium (Spur 1) wird außer der Transglutaminase mit einer Molmasse von 38 kDa eine weitere Proteinbande bei ca. 45 kDa angefärbt. Durch weitergehende Charakterisierung konnte sowohl auf Protein- als auch auf Nukleinsäureebene gezeigt werden, daß es sich bei diesem kreuzreaktiven Protein um das inaktive Pro-Enzym der bakteriellen Transglutaminase handelt. Aus einem zusätzlichen N-terminalen Peptid resultiert das höhere Molekulargewicht.

### 9. Überprüfung der Substrateigenschaften der Transglutaminase-Tag-Peptidsequenz DEQQSA

### a) Herstellung einer 14,8 mM DEQQSA-Lösung

10 mg Peptid mit der Sequenz DEQQSA werden in 1 ml bidest. Wasser gelöst.

### b) Herstellung einer 14,8 mM C-DNS-Lösung

11,5 mg Dansylcadaverin werden in 10 µl konz. HCl gelöst und mit 2,31 ml einer DMSO-Lösung, die aus 0,01 M HCl und DMSO im Verhältnis 1 : 1 (v/v) hergestellt wird, verdünnt.

### c) Enzymatischer Einbau von Dansylcadaverin in das Peptid DEQQSA

10 µl DEQQSA-Lösung und 10 µl C-DNS-Lösung werden mit 70 µl 1 M Tris-HCl-Puffer gemischt. Nach Zugabe von 10 µl Transglutaminase der Aktivität 16 E/ml inkubiert man zur vollständigen Umsetzung 3 h bei 37 ° C.

### d) HPLC-Nachweis

10 µl der Inkubationsmischung werden mit 40 µl Trifluoressigsäure (TFA) gemischt. 10 µl der TFA-Lösung werden auf einer 5 x 300 mm Nucleosil RP₁₈-Säule (Macherey & Nagel) mit Acetonitril, das 0,1 % TFA enthält, getrennt. Das Peptid DEQQSA besitzt eine Retentionszeit von 4,97 min, das Hauptprodukt mit einem Flächenanteil von ca. 76 % erscheint nach 8,41 min, das Nebenprodukt mit einem Flächenanteil von 24 % nach 8,65 min.

## Patentansprüche

1. Transglutaminase-Polypeptidmolekül gemäss der Formel:
R₁-R₂-R₃
wobei
R₁ entweder Wasserstoff, Methionin oder eine Aminosäure-Pre-Sequenz bedeutet;
R₂ eine Aminosäure-Pro-Sequenz von *Streptoverticillium mobaraense* bedeutet, wobei R₂ die folgende Sequenz hat: und
R₃ eine Aminosäure-Sequenz für ein aktives bakterielles Transglutaminase-Polypeptid aus *Streptoverticillium mobaraense* bedeutet, wobei R₃ die folgende Sequenz hat:

2. Transglutaminase-Polypeptidmolekül nach Anspruch 1, wobei R₁ eine prokaryontische Pre-Sequenz ist.

3. Transglutaminase-Polypeptidmolekül nach Anspruch 2, wobei R₁ eine Pre-Sequenz aus Actinomyceten ist.

4. Transglutaminase-Polypeptidmolekül nach Anspruch 3, wobei R₁ eine Pre-Sequenz von Streptoverticillium, bevorzugt entweder eine Pre-Sequenz von *Streptoverticillium mobaraense* oder eine Pre-Sequenz von *Streptoverticillium fervens subsp. melrosporus* ist.

5. Transglutaminase-Nukleinsäuremolekül gemäss der Formel:
N₁-N₂-N₃
wobei
N₁ eine 5'-Phosphatgruppe, eine 5'-OH-Gruppe oder eine Nukleinsäuresequenz bedeutet, die für Methionin oder eine Pre-Sequenz kodiert;
N₂ eine Nukleinsäuresequenz bedeutet, die für eine Pro-Sequenz aus *Streptoverticillium mobaraense* kodiert, wobei N₂ die folgende Sequenz umfasst: und
N₃ eine Nukleinsäuresequenz bedeutet, die für ein aktives bakterielles Transglutaminase-Polypeptidmolekül aus *Streptoverticillium mobaraense* kodiert, wobei N₃ die folgende Sequenz umfasst:

6. Transglutaminase-Nukleinsäuremolekül nach Anspruch 5, wobei N₁ für eine Pre-Sequenz aus Actinomyceten kodiert.

7. Transglutaminase-Nukleinsäuremolekül nach Anspruch 6, wobei N1 für eine Pre-Sequenz aus Streptoverticillium, bevorzugt entweder für eine Pre-Sequenz aus *Streptoverticillium mobaraense* oder für eine Pre-Sequenz aus *Streptoverticillium fervens subsp. melrosporus* kodiert.

8. Expressionsvektor, der ein Nukleinsäuremolekül nach einem der Ansprüche 5 bis 7 enthält.

9. Wirtsorganismus, der einen Expressionsvektor nach Anspruch 8 enthält.

10. Wirtsorganismus nach Anspruch 9, wobei der Wirtsorganismus ein Gramnegativer oder Gram-positiver prokaryontischer Wirtsorganismus ist.

11. Wirtsorganismus nach Anspruch 10, wobei der Gram-negative prokaryontische Wirtsorganismus *Escherichia coli* ist.

12. Wirtsorganismus nach Anspruch 10, wobei der Gram-positive prokaryontische Wirtsorganismus ein Actinomycet, bevorzugt *Streptomyces lividans*, ist.

13. Wirtsorganismus nach einem der Ansprüche 9 bis 12, wobei der Wirtsorganismus keine oder nur unzureichende Mengen einer aktiven Protease exprimiert, die zur Abspaltung der Pro-Sequenz R₂ von R₃ fähig ist.

14. Verfahren zum Herstellen eines inaktiven Pro-Polypeptidmoleküls einer Transglutaminase nach einem der Ansprüche 1 bis 4 durch Kultivieren eines Wirtsorganismus nach Anspruch 9 bis 12, wobei der in dem Wirtsorganismus enthaltene Expressionsvektor ein Nukleinsäuremolekül nach einem der Ansprüche 5 bis 7 enthält, und Ernten des Pro-Polypeptidmoleküls aus dem Kulturüberstand und/oder den Zellen.

15. Verfahren zum Aktivieren eines Transglutaminase-Pro-Polypeptidmoleküls nach mindestens einem der Ansprüche 1 bis 4 durch enzymatische, chemische oder physikalische Spaltung.

16. Verfahren nach Anspruch 15, wobei die enzymatische Spaltung mit der Protease Dispase durchgeführt wird.

17. Verfahren nach Anspruch 15, wobei die enzymatische Spaltung mit der Protease Trypsin durchgeführt wird.

18. Verfahren nach Anspruch 15, wobei die enzymatische Spaltung mit der Protease Chymotrypsin durchgeführt wird.

19. Verfahren zum kovalenten Verknüpfen von Proteinen und/oder Peptiden unter Verwendung eines Transglutaminase-Polypeptidmoleküls aus Streptoverticillium gemäß einem der Ansprüche 1 bis 4, wobei die Proteine und/oder Peptide unter geeigneten Versuchsbedingungen mit dem Transglutaminase-Pro-Polypeptidmolekül in Kontakt gebracht werden und zu einem gewählten Zeitpunkt aktiviert werden.

## Claims

1. Transglutaminase polypeptide molecule according to the formula:
R₁-R₂-R₃
wherein
R₁ means either hydrogen, methionine or an amino acid pre-sequence;
R₂ means an amino acid pro-sequence of *Streptoverticillium mobaraense* wherein R₂ has the following sequence: and
R₃ means an amino acid sequence for an active, bacterial transglutaminase polypeptide from *Streptoverticillium mobaraense,* wherein R₃ has the following sequence:

2. Transglutaminase polypeptide molecule according to claim 1, wherein R₁ is a procaryotic pre-sequence.

3. Transglutaminase polypeptide molecule according to claim 2, wherein R₁ is a pre-sequence of actinomycetes.

4. Transglutaminase polypeptide molecule according to claim 3, wherein R₁ is a pre-sequence of Streptoverticillium, preferably either a pre-sequence of *Streptoverticillium mobaraense* or a pre-sequence of *Streptoverticillium fervens subsp. melrosporus*.

5. Transglutaminase nucleic acid molecule according to the formula:
N₁-N₂-N₃
wherein
N₁ means a 5'-phosphate group, a 5'-OH group or a nucleic acid sequence, which encodes for methionine or a pre-sequence;
N₂ means a nucleic acid sequence, which encodes for a pro-sequence of *Streptoverticillium mobaraense,* wherein N₂ comprises the following sequence: and
N₃ means a nucleic acid sequence, which encodes for an active, bacterial transglutaminase polypeptide molecule from *Streptoverticillium mobaraense,* wherein N₃ comprises the following sequence:

6. Transglutaminase nucleic acid molecule according to claim 5, wherein N₁ encodes for a pre-sequence of actinomycetes.

7. Transglutaminase nucleic acid molecule according to claim 6, wherein N₁ encodes for a pre-sequence of Streptoverticillium, preferably either for a pre-sequence of *Streptoverticillium mobaraense* or for a pre-sequence of *Streptoverticillium fervens subsp. melrosporus.*

8. Expression vector containing a nucleic acid molecule according to one of the claims 5 to 7.

9. Host organism containing an expression vector according to claim 8.

10. Host organism according to claim 9, wherein the host organism is a Gram-negative or Gram-positive procaryotic host organism.

11. Host organism according to claim 10, wherein the Gram-negative, procaryotic host organism is *Escherichia coli*.

12. Host organism according to claim 10, wherein the Gram-positive, procaryotic host organism is an actinomycete, preferably *Streptomyces lividans*.

13. Host organism according to one of the claims 9 to 12, wherein the host organism doesn't express at all, or only in insufficient quantities, an active protease capable of performing the cleavage of the pro-sequence R₂ from R₃.

14. Method for producing an inactive pro-polypeptide molecule of a transglutaminase according to one of the claims 1 to 4 by cultivating a host organism according to claims 9 to 12, wherein the expression vector comprised in the host organism contains a nucleic acid molecule according to one of the claims 5 to 7, and for harvesting the pro-polypeptide molecule from the culture supernatant and/ or from the cells.

15. Method for activating a transglutaminase-pro-polypeptide molecule according to at least one of the claims 1 to 4 by enzymatic, chemical or physical cleavage.

16. Method according to claim 15 wherein the enzymatic cleavage is achieved by the protease dispase.

17. Method according to claim 15 wherein the enzymatic cleavage is achieved by the protease trypsin.

18. Method according to claim 15 wherein the enzymatic cleavage is achieved by the protease chymotrypsin.

19. Method for covalent linking of proteins and/ or peptides using a transglutaminase polypeptide molecule from Streptoverticillium according to one of the claims 1 to 4, wherein the proteins and/ or peptides are exposed to the transglutaminase pro-polypeptide molecule under adequate test conditions and are activated at a chosen moment.

## Revendications

1. Molécule de pro-polypeptide transglutaminase selon la formule :
R₁-R₂-R₃
où
R₁ signifie ou hydrogène, méthionine ou bien une pré-séquence d'acides aminés ;
R₂ signifie une pro-séquence, où R₂ a la séquence suivante : et
R₃ signifie une séquence d'acide aminé pour un polypeptide actif, bactérien transglutaminase de *Streptoverticillium mobaraense*, où R₃ a la séquence suivante :

2. Molécule de polypeptide transglutaminase selon la revendication 1, où R₁ est une pré-séquence procaryotique.

3. Molécule de polypeptide transglutaminase selon la revendication 2, où R₁ est une préséquence d'actinomycètes.

4. Molécule de polypeptide transglutaminase selon la revendication 3, où R₁ est une pré-séquence de *Streptoverticillium,* de préférence une pré-séquence de *Streptoverticillium mobaraense* ou bien une pré-séquence de *Streptoverticillium fervens subsp. mobaraense.*

5. Molécule de transglutaminase et acide nucléique selon la formule :
N₁ - N₂ - N₃
où
N₁ représente un 5' groupe phosphate, un 5' groupe OH ou bien une séquence d'acide nucléique codant pour methionine ou une pré-séquence;
N₂ représente une séquence d'acide nucléique codant pour une pro-séquence de *Streptoverticillium mobaraense*, où N₂ comprend la séquence suivante : et
N₃ représente une séquence d'acide nucléique codant pour une molécule active et bactérienne de polypeptide transglutaminase dérivant de *Streptoverticillium mobaraense* où N3 comprend la séquence suivante :

6. Molécule d'acide nucléique transglutaminase selon la revendication 5, où N₁ code pour une pré-séquence d'actinomycètes.

7. Molécule d'acide nucléique transglutaminase selon la revendication 6 où N₁ code pour une pré-séquence de Streptoverticillium, de préférence ou pour une pré-séquence de *Streptoverticillium mobaraense* ou pour une pré-séquence de *Streptoverticillium fervens subsp. melrosporus.*

8. Vecteur d'expression, lequel contient une molécule selon l'une quelconque des revendications 5 à 7.

9. Organisme hôte, lequel contient un vecteur d'expression selon la revendication 8.

10. Organisme hôte selon la revendication 9, où l'organisme hôte est un organisme hôte procaryotique à Gram positif ou à Gram négatif.

11. Organisme hôte selon la revendication 10, où l'organisme hôte procaryotique et Gram négative est *Escherichia coli.*

12. Organisme hôte selon la revendication 10, où l'organisme hôte procaryotique est Gram positive et une actinomycète, de préférence *Streptomyces lividans*.

13. Organisme hôte selon l'une quelconque des revendication 9 à 12, où l'organisme hôte n'exprime pas, ou seulement dans une quantité insuffisante, une protéase active, laquelle peut effectuer le clivage de la pro-séquence R₂ de R₃.

14. Méthode pour produire une molécule inactive de pro-polypeptide d'une transglutaminase selon l'une quelconque des revendications 1 à 4 en cultivant un organisme hôte selon la revendication 9 à 12, où le vecteur d'expression compris dans l'organisme hôte contient une molécule d'acide nucléique selon l'une quelconque des revendication 5 à 7 et en recueillant de la molécule de pro-polypeptide du surnageant et/ou des cellules.

15. Méthode pour activer une molécule de transglutaminase pro-polypeptide selon au moins l'une des revendications 1 à 4 par clivage enzymatique, chimique ou physique.

16. Méthode selon la revendication 15, où le clivage enzymatique est procédé par la protéase dispase.

17. Méthode selon la revendication 15, où le clivage enzymatique est procédé par la protéase trypsine.

18. Méthode selon la revendication 15, où le clivage enzymatique est procédé par la protéase chymotrypsine.

19. Méthode pour effectuer une liaison covalente entre les protéines et/ou peptides en utilisant une molécule de polypeptide transglutaminase dérivant de Streptoverticillium selon l'une quelconque des revendications 1 à 4 où les protéines et/ou les peptides sont exposés au contact avec la molécule de transglutaminase pro-polypeptide dans des conditions expérimentales appropriées et activés à un moment choisi.
